# EUROPÄISCHE PATENTSCHRIFT

(11) **EP 1 809 654 B1**
(45) Veröffentlichungstag und Bekanntmachung des Hinweises auf die Patenterteilung: **08.10.2008**
(21) Anmeldenummer: 05796321.7
(22) Anmeldetag: 08.10.2005
(51) Int. Cl.: C07K 7/06, A61K 38/08, A61P 31/04

(54) **HETEROCYCLYL-SUBSTITUIERTE NONADEPSIPEPTIDE**
HETEROCYCLYL-SUBSTITUTED NONADEPSIPEPTIDES
NONADEPSIPEPTIDES A SUBSTITUTION HETEROCYCLYLE

(30) Priorität: 20.10.2004 DE 102004051024
(43) Veröffentlichungstag der Anmeldung: 25.07.2007
(73) Patentinhaber: AiCuris GmbH & Co. KG, 42117 Wuppertal (DE)
(72) Erfinder: VON NUSSBAUM, Franz, 40219 Düsseldorf (DE); BRUNNER, Nina, 45279 Essen (DE); ENDERMANN, Rainer, 42113 Wuppertal (DE); FÜRSTNER, Chantal, 45478 Mühlheim/Ruhr (DE); HARTMANN, Elke, 42111 Wuppertal (DE); RAGOT, Jacques, 40625 Düsseldorf (DE); SCHIFFER, Guido, 42111 Wuppertal (DE); SCHUHMACHER, Joachim, 42113 Wuppertal (DE); SVENSTRUP, Niels, 42553 Velbert (DE); TELSER, Joachim, 51061 Köln (DE); ANLAUF, Sonja, 42115 Wuppertal (DE); BRÜNING, Michael-Alexander, 13469 Berlin (DE)
(74) Vertreter: Weller, Wolfgang
(86) Internationale Anmeldenummer: PCT/EP2005/010856
(87) Internationale Veröffentlichungsnummer: WO 2006/042653

(56) Entgegenhaltungen:
- WO-A-20/04099239
- TYMIAK A A ET AL: "STRUCTURE DETERMINATION OF LYSOBACTIN A MACROCYCLIC PEPTIDE LACTONE ANTIBIOTIC" JOURNAL OF ORGANIC CHEMISTRY, AMERICAN CHEMICAL SOCIETY. EASTON, US, Bd. 54, Nr. 5, 1989, Seiten 1149-1157, XP002289533 ISSN: 0022-3263 in der Anmeldung erwähnt
- EGNER B J ET AL: "Monitoring the solid phase synthesis of analogues of lysobactin and the katanosins using in situ MALDI-TOF MS" TETRAHEDRON, ELSEVIER SCIENCE PUBLISHERS, AMSTERDAM, NL, Bd. 53, Nr. 41, 13. Oktober 1997 (1997-10-13), Seiten 14021-14030, XP004618631 ISSN: 0040-4020

## Beschreibung

Die Erfindung betrifft Nonadepsipeptide und Verfahren zu ihrer Herstellung sowie ihre Verwendung zur Herstellung von Arzneimitteln zur Behandlung und/oder Prophylaxe von Krankheiten, insbesondere bakteriellen Infektionskrankheiten.

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine sind innerhalb der Bakterien stark konserviert. Aufgrund ihrer essentiellen Natur und Einheitlichkeit ist die Zellwandbiosynthese ein idealer Angriffspunkt für neue Antibiotika (D.W.Green, The bacterial cell wall as a source of antibacterial targets, Expert Opin. Ther. Targets, 2002, 6, 1-19).

Vancomycin und Penicilline sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen, vor allem mit Grampositiven Erregern eingesetzt. Durch das wachsende Auftreten von resistenten Keimen, z.B. Methicillin-resistenten Staphylokokken, Penicillin-resistenten Pneumokokken und Vancomycinresistenten Enterokokken (F. Baquero, Gram-positive resistance: challenge for the development of new antibiotics, J. Antimicrob. Chemother., 1997, 39, Suppl A:1-6; A.P. Johnson, D.M. Livermore, G.S. Tillotson, Antimicrobial susceptibility of Gram-positive bacteria: what's current, what's anticipated ?, J. Hosp. Infect., 2001, (49), Suppl A: 3-11) sowie jüngst auch erstmals Vancomycinresistenten Staphylokokken (B. Goldrick, First reported case of VRSA in the United States, Am. J. Nurs., 2002, 102, 17) verlieren diese Substanzen zunehmend ihre therapeutische Wirksamkeit.

Die vorliegende Erfindung beschreibt eine neue Klasse von Zellwandbiosynthese-Inhibitoren ohne Kreuzresistenzen zu bekannten Antibiotika-Klassen.

Der Naturstoff Lysobactin und einige Derivate sind als antibakteriell wirksam beschrieben in US 4,754,018. Die Isolierung und antibakterielle Wirksamkeit von Lysobactin ist auch in EP-A 196 042 und JP 01132600 beschrieben. WO04/099239 beschreibt Derivate des Lysobactins mit antibakterieller Wirksamkeit.

Die antibakterielle Wirkung von Lysobactin und Katanosin A wird weiterhin beschrieben in O'Sullivan, J. et al., J. Antibiot. 1988, 41, 1740 - 1744, Bonner, D. P. et al., J. Antibiot. 1988, 41, 1745 - 1751, Shoji, J. et al., J. Antibiot. 1988, 41, 713 - 718 Tymiak, A. A. et al., J. Org. Chem. 1989, 54, 1149 - 1157 und Egner, B.J. et al., Tetrahedron 1997, 53, 14021 - 14030.

Eine Aufgabe der vorliegenden Erfindung ist es, alternative Verbindungen mit vergleichbarer oder verbesserter antibakterieller Wirkung und besserer Verträglichkeit, z.B. geringerer Nephrotoxizität, zur Behandlung von bakteriellen Erkrankungen bei Menschen und Tieren zur Verfügung zu stellen.

Gegenstand der Erfindung sind Verbindungen der Formel 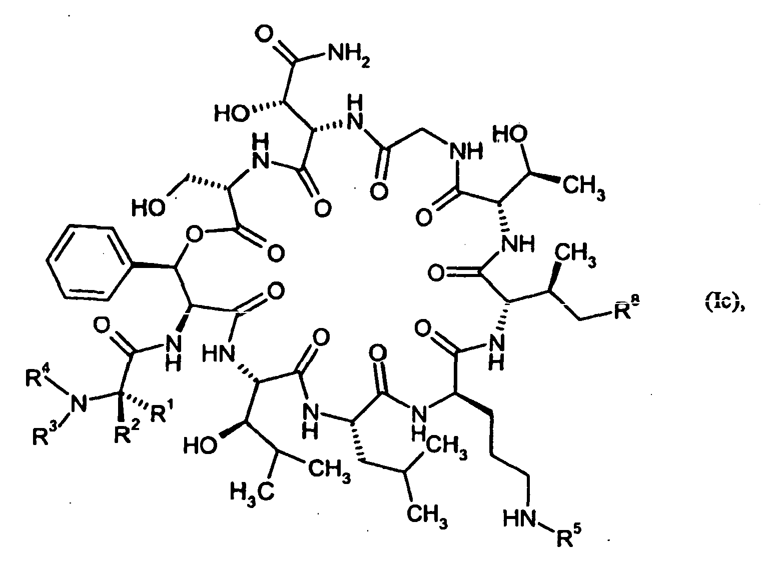 in welcher
- R¹: Wasserstoff, C₁-C₆-Alkyl, C₂-C₆-Alkenyl, C₃-C₆-Cycloalkyl oder C₆-C₁₀-Aryl bedeutet,
wobei Alkyl, Alkenyl, Cycloalkyl und Aryl substituiert sein können mit 0, 1, 2 oder 3 Substituenten unabhängig voneinander ausgewählt aus der Gruppe bestehend aus Halogen, Hydroxy, Amino, Cyano, Trimethylsilyl, C₁-C₆-Alkyl, C₁-C₆-Alkoxy, Benzyloxy, C₃-C₆-Cycloalkyl, C₆-C₁₀-Aryl, 5- bis 7-gliedriges Heterocyclyl, 5- bis 10-gliedriges Heteroaryl, C₁-C₆-Alkylamino, C₆-C₁₀-Arylamino, C₁-C₆-Alkylcarbonylamino, C₆-C₁₀-Arylcarbonylamino, C₁-C₆-Alkylcarbonyl, C₁-C₆-Alkoxycarbonyl, C₆-C₁₀-Arylcarbonyl und Benzyloxycarbonylamino,
worin Cycloalkyl, Aryl, Heterocyclyl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Trifluormethyl, C₁-C₆-Alkyl, C₁-C₆-Alkoxy, Phenyl und 5- bis 7-gliedriges Heterocyclyl,
- R²: Wasserstoff oder C₁-C₄-Alkyl bedeutet,
- R³: C₁-C₆-Alkyl, C₃-C₆-Cycloalkyl, 5- bis 7-gliedriges Heterocyclyl, C₆-C₁₀-Aryl, 5- oder 6-gliedriges Heteroaryl, C₁-C₆-Alkylcarbonyl, C₁-C₆-Alkoxycarbonyl, C₃-C₆-Cycloalkylcarbonyl, 5- bis 7-gliedriges Heterocyclylcarbonyl, C₆-C₁₀-Arylcarbonyl, 5- oder 6-gliedriges Heteroarylcarbonyl oder C₁-C₆-Alkylaminocarbonyl bedeutet,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, C₁-C₆-Alkylamino und Phenyl,
und
wobei Alkylcarbonyl substituiert ist mit einem Substituenten Amino oder C₁-C₆-Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, C₁-C₆-Alkoxy, C₁-C₆-Alkylthio, Benzyloxy, C₃-C₆-Cycloalkyl, Phenyl, Naphthyl, 5- bis 10-gliedriges Heteroaryl, C₁-C₆-Alkylcarbonylamino, C₁-C₆-Alkoxycarbonylamino, C₆-C₁₀-Arylcarbonylamino, C₆-C₁₀-Arylearbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, C₁-C₆-Alkyl, C₁-C₆-Alkoxy und Phenyl,
- R⁴: Wasserstoff, C₁-C₄-Alkyl, Cyclopropyl oder Cyclopropylmethyl bedeutet,
- R⁵: eine Gruppe der Formel 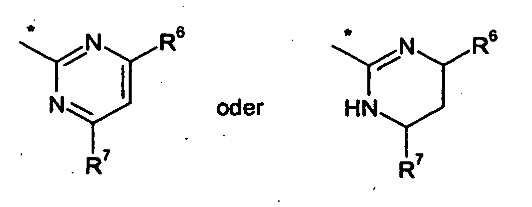 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
- R⁸: Wasserstoff oder Methyl bedeutet,
und ihre Salze, ihre Solvate und die Solvate ihrer Salze.

Erfindungsgemäße Verbindungen sind die Verbindungen der Formel (I) und (Ic) und deren Salze, Solvate, und Solvate der Salze, die von Formel (I) und (Ic) umfassten Verbindungen der nachfolgend genannten Formeln und deren Salze, Solvate und Solvate der Salze sowie die von Formel (I) und (Ic) umfassen, nachfolgend als Ausführungsbeispiele genannten Verbindungen und deren Salze, Solvate, und Solvate der Salze, soweit es sich bei den von Formel (I) und (Ic) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können oder Mischsalze.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyctohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylthio, Alkylcarbonyl, Alkoxycarbonyl, Alkylaminocarbonyl, Alkylcarbonylamino und Alkoxycarbonylamino steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert-*Butyl, 2,2-Dimethylprop-1-yl, 2,2-Dimethylbut-1-yl, n-Pentyl und n-Hexyl.
Alkoxy steht beispielhaft und vorzugsweise für Methoxy Ethoxy, n-Propoxy, Isopropoxy, *tert-*Butoxy, n-Pentoxy und n-Hexoxy.
Alkylthio steht beispielhaft und vorzugsweise für Methylthio, Ethylthio, n-Propylthio, Isopropylthio, *tert*-Butylthio, n-Pentylthio und n-Hexylthio.
Alkenyl steht für einen geradkettigen oder verzweigten Alkenylrest mit 2 bis 6 Kohlenstoffatomen. Bevorzugt ist ein geradkettiger oder verzweigter Alkenylrest mit 2 bis 4, besonders bevorzugt mit 2 bis 3 Kohlenstoffatomen. Beispielsweise und vorzugsweise seien genannt: Vinyl, Allyl, n-Prop-1-en-1-yl, n-But-2-en-1-yl, 2-Methylprop-1-en-1-yl und 2-Methylprop-2-en-1-yl.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N,N*-Dimethylamino, *N,N-*Diethylamino, *N*-Ethyl-*N*-methylamino, *N*-Methyl-*N-*n-propylamino, *N*-Isopropyl-*N*-n-propylamino, *N*-*tert*-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Arylamino steht für einen über eine Aminogruppe gebundenen Arylsubstituenten, wobei an die Aminogruppe gegebenenfalls ein weiterer Substituenten, wie z.B. Aryl oder Alkyl, gebunden ist, beispielhaft und vorzugsweise für Phenylamino, Naphthylamino, Phenylmethylamino oder Diphenylamino.
Alkylcarbonyl steht beispielhaft und vorzugsweise für Methylcarbonyl, Ethylcarbonyl, n-Propylcarbonyl, Isopropylcarbonyl, *tert*-Butylcarbonyl, n-Pentylcarbonyl und n-Hexylcarbonyl.
Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.
Alkoxycarbonylamino steht beispielhaft und vorzugsweise für Methoxycarbonylamino, Ethoxycarbonylamino, n-Propoxy-carbonylamino, Isopropoxycarbonylamino, *tert*-Butoxycarbonyl-amino, n-Pentoxycarbonylamino und n-Hexoxycarbonylamino.
Cycloalkylcarbonyl steht für einen über eine Carbonylgruppe gebundenen Cycloalkylsubstituenten, beispielhaft und vorzugsweise für Cyclopropylcarbonyl, Cyclobutylcarbonyl, Cyclopentylcarbonyl und Cyclohexylcarbonyl.
Heterocyclylcarbonyl steht für einen über eine Carbonylgruppe gebundenen Heterocyclylsubstituenten, beispielhaft und vorzugsweise für Tetrahydrofuranylcarbonyl, Pyrrolidinylcarbonyl, Pyrrolinylcarbonyl, Piperidinylcarbonyl, Tetrahydropyranylcarbonyl, Piperazinylcarbonyl, Morpholinylcarbonyl und Perhydroazepinylcarbonyl.
Arylcarbonyl steht für einen über eine Carbonylgruppe gebundenen Arylsubstituenten, beispielhaft und vorzugsweise für Phenylcarbonyl, Naphthylcarbonyl und Phenanthrenylcarbonyl.
Hetercarylcarbonyl steht für einen über eine Carbonylgruppe gebundenen Heteroarylsubstituenten, beispielhaft und vorzugsweise für Thienylcarbonyl, Furylcarbonyl, Pyrrolylcarbonyl, Thiazolylcarbonyl, Oxazolylcarbonyl, Imidazolylcarbonyl, Pyridylcarbonyl, Pyrimidylcarbonyl, Pyridazinylcarbonyl, Indolylcarbonyl, Indazolylcarbonyl, Benzofuranylcarbonyl, Benzothiophenyl-carbonyl, Chinolinylcarbonyl und Isochinolinylcarbonyl.
Alkylcarbonylamino steht beispielhaft und vorzugsweise für Methylcarbonylamino, Ethylcarbonylamino, n-Propylcarbonylamino, Isopropylcarbonylamino, *tert*-Butylcarbonylamino, n-Pentylcarbonylamino und n-Hexylcarbonylamino.
Arylcarbonylamino steht beispielhaft und vorzugsweise für Phenylcarbonylamino, Naphthylcarbonylamino und Phenanthrenylcarbonylamino.
Arylcarbonyloxy steht beispielhaft und vorzugsweise für Phenylcarbonyloxy, Naphthylcarbonyloxy und Phenanthrenylcarbonyloxy.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, Isopropylaminocarbonyl, *tert*-Butylaminocarbonyl, n-Pentylaminocarbonyl, n-Hexylaminocarbonyl, *N,N*-Dimethylaminocarbonyl, *N,N*-Diethylaminocarbonyl, *N-*Ethyl-*N-*methylaminocarbonyl, *N*-Methyl-*N-*n-propylaminocarbonyl, *N-*Isopropyl-*N*-n-propylaminocarbonyl, *N-tert*-Butyl-*N-*methylaminocarbonyl, *N-*Ethyl-*N-*n-pentylamino-carbonyl und *N-*n-Hexyl-*N-*methylaminocarbonyl. C₁-C₃-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent.
Cycloalkyl steht für eine Cycloalkylgruppe mit in der Regel 3 bis 6 Kohlenstoffatomen, beispielhaft und vorzugsweise für Cyclopropyl, Cyclobutyl, Cyclopentyl und Cyclohexyl.
Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.
Heterocyclyl steht für einen mono- oder polycyclischen, vorzugsweise mono- oder bicyclischen, heterocyclischen Rest mit in der Regel 5 bis 7 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- bis 7-gliedrige, monocyclische gesättigte Heterocyclylreste mit bis zu zwei Heteroatomen aus der Reihe O, N und S, wie beispielhaft und vorzugsweise Tetrahydrofuranyl, Pyrrolidinyl, Pyrrolinyl, Piperidinyl, Tetrahydropyranyl, Piperazinyl, Morpholinyl und Perhydroazepinyl.
Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl und Isochinolinyl.
Halogen steht für Fluor, Chlor, Brom und Jod, bevorzugt Fluor und Chlor.

### Beschreibung der Abbildungen

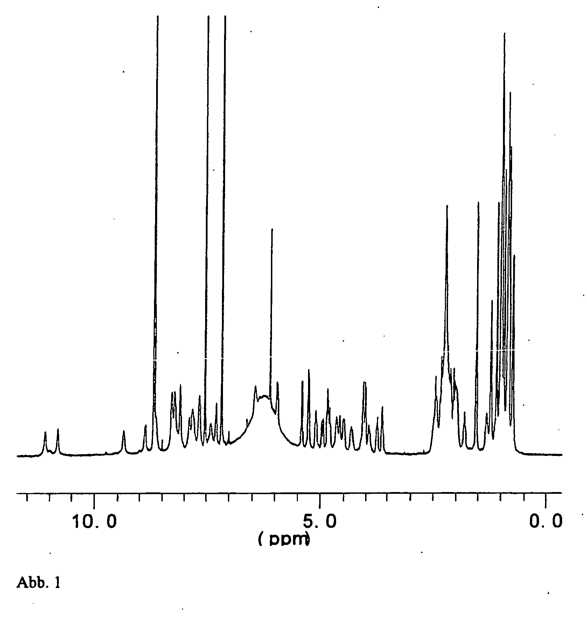

Abb. 1: ¹H-NMR von *N*^{ω.*6*},*N*^{ω'.*6*}-(Pent[2]en[2]yl[4]yliden)-lysobactin-trifluoracetat (*d₅*-Pyridin, 500 MHz).

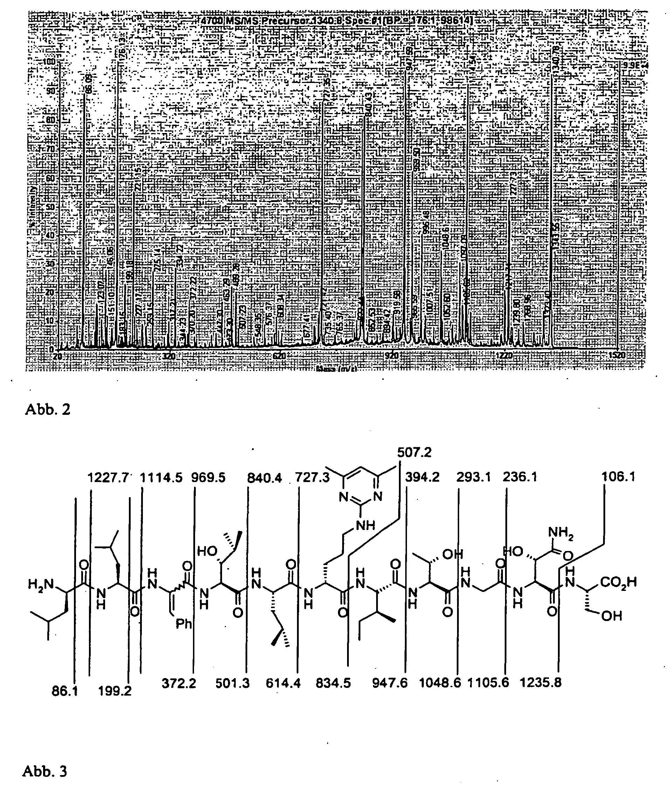

Abb. 2: MALDI-MS/MS-Spektrum (positive Ionen, Vorläuferion *m*/*z* 1340.8) von *N*^{ω.6},*N*^{ω'.6}-(Pent[2]en[2]yl[4]yliden)-lysobactin-trifluoracetat.
Abb. 3: Fragmente des MALDI-MS/MS-Spektrums (positive Ionen, Vorläuferion *m*/*z* 1340.8) von *N*^{ω.6},*N*^{ω'.6}-(Pent[2]en[2]yl[4]yliden)-lysobactin-trifluoracetat.

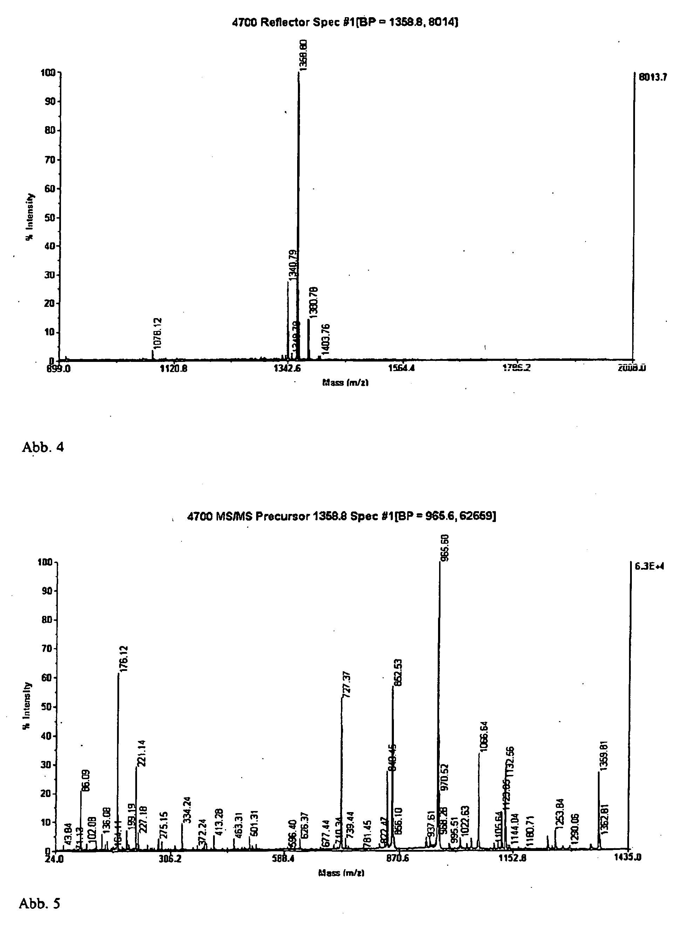

Abb. 4: MALDI-MS-Spektrum (positive Ionen) von hydrolytisch Ring-geöffnetem *N*^{ω}*^{6.},N*^{ω}*^{'.6}-*(Pent[2]en[2]yl[4]yliden)-lysobactin-trifluoracetat.
Abb. 5: MALDI-MS/MS-Spektrum (positive Ionen, Vorläuferion *m*/*z* 1358.8) von hydrolytisch Ring-geöffnetem *N*^{ω.*6*},*N*^{ω'.*6*}-(Pent[2]en[2]yl[4]yliden)-lysobactin-trifluoracetat.

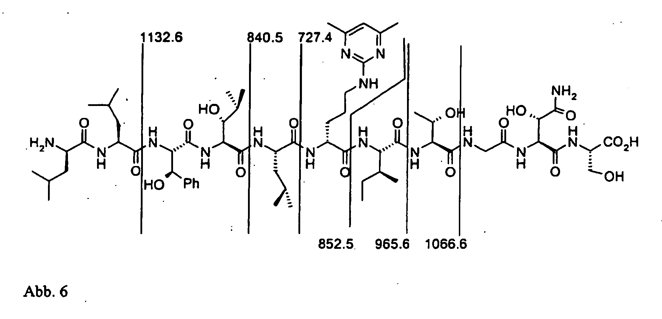

Abb. 6: Fragmente des MALDI-MS/MS-Spektrums (positive Ionen, Vorläuferion *m*/*z* 1358.8) von hydrolytisch Ring-geöffnetem *N*^{ω.*6*},*N*^{ω.*'6*} -(Pent[2]en[2]yl[4]yliden)-lysobactin-trifluoracetat.

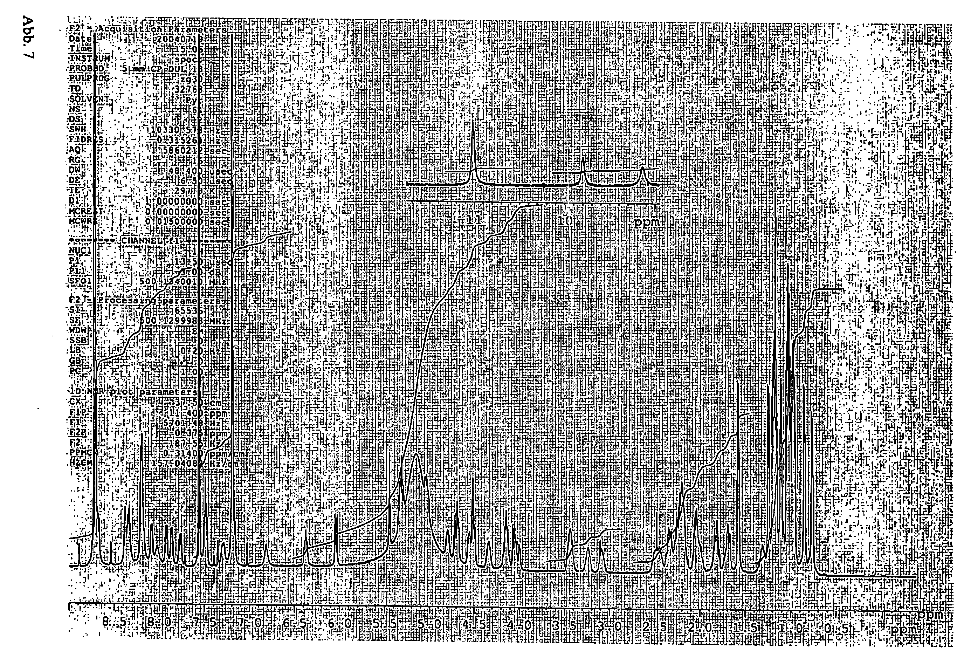

Abb. 7: ¹H-NMR von *N*^{ω.*6*},*N*^{ω.*'6*}-(Pentan[2,4]diyl)-lysobactin-trifluoracetat (*d₅*-Pyridin, 500 MHz).

Bevorzugt im Rahmen der vorliegenden Erfindung sind Verbindungen der Formel 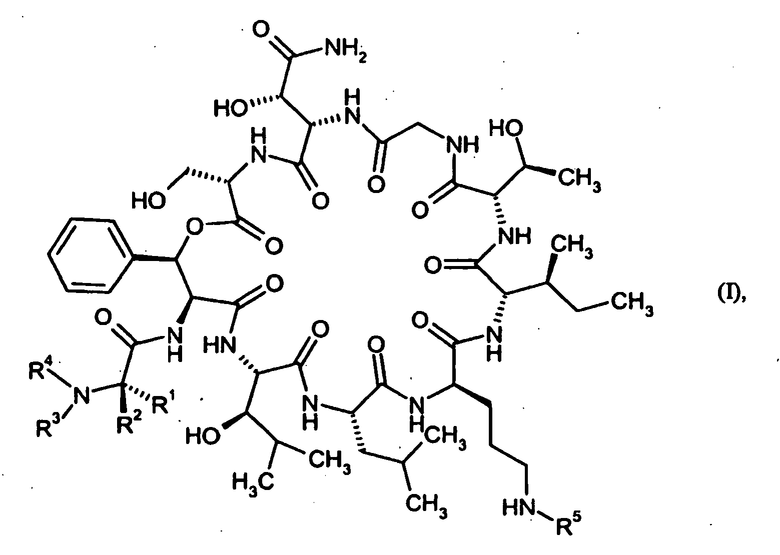 in welcher
- R¹: Wasserstoff, C₁-C₆-Alkyl, C₂-C₆-Alkenyl, C₃-C₆-Cycloalkyl oder C₆-C₁₀-Aryl bedeutet,
wobei Alkyl, Alkenyl, Cycloalkyl und Aryl substituiert sein können mit 0, 1, 2 oder 3 Substituenten unabhängig voneinander ausgewählt aus der Gruppe bestehend aus Halogen, Hydroxy, Amino, Cyano, Trimethylsilyl, C₁-C₆₋Alkyl, C₁-C₆-Alkoxy, Benzyloxy, C₃-C₆-Cycloalkyl, C₆-C₁₀-Aryl, 5- bis 7-gliedriges Heterocyclyl, 5- bis 10-gliedriges Heteroaryl, C₁-C₆-Alkylamino, C₆-C₁₀-Arylamino, C₁-C₆-Alkylcarbonylamino, C₆-C₁₀-Arylcarbonylamino, C₁-C₆-Alkylcarbonyl, C₁-C₆-Alkoxycarbonyl, C₆-C₁₀-Arylcarbonyl und Benzyloxycarbonylamino,
worin Cycloalkyl, Aryl, Heterocyclyl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Trifluormethyl, C₁-C₆-Alkyl, C₁-C₆-Alkoxy, Phenyl und 5- bis 7-gliedriges Heterocyclyl,
- R²: Wasserstoff oder C₁-C₄-Alkyl bedeutet,
- R³: C₁-C₆-Alkyl, C₃-C₆-Cycloalkyl, 5- bis 7-gliedriges Heterocyclyl, C₆-C₁₀-Aryl, 5- oder 6-gliedriges Heteroaryl, C₁-C₆-Alkylcarbonyl, C₁-C₆-Alkoxycarbonyl, C₃-C₆-Cycloalkylcarbonyl, 5- bis 7-gliedriges Heterocyclylcarbonyl, C₆-C₁₀-Arylcarbonyl, 5- oder 6-gliedriges Heteroarylcarbonyl oder C₁-C₆-Alkylaminocarbonyl bedeutet,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, C₁-C₆-Alkylamino und Phenyl,
und
wobei Alkylcarbonyl substituiert ist mit einem Substituenten Amino oder C₁-C₆-Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, C₁-C₆-Alkoxy, C₁-C₆-Alkylthio, Benzyloxy, C₃-C₆-Cycloalkyl, Phenyl, Naphthyl, 5- bis 10-gliedriges Heteroaryl, C₁-C₆-Alkylcarbonylamino, C₁-C₆-Alkoxycarbonylamino, C₆-C₁₀-Arylcarbonylamino, C₆-C₁₀-Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, C₁-C₆-Alkyl, C₁-C₆-Alkoxy und Phenyl,
- R⁴: Wasserstoff, C₁-C₄-Alkyl, Cyclopropyl oder Cyclopropylmethyl bedeutet,
- R⁵: eine Gruppe der Formel 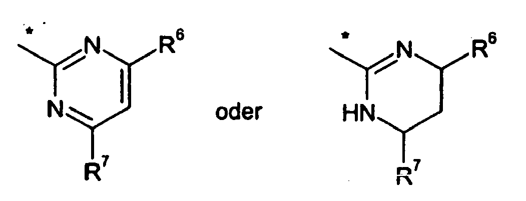 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
und ihre Salze, ihre Solvate und die Solvate ihrer Salze.

Bevorzugt sind auch Verbindungen der Formel (I), in welcher
- R¹: 2-Methylprop-1-yl, 2,2-Dimethylprop-1-yl, 2,2-Dimethylbut-1-yl, 1-Trimethylsilylmethyl, 2-Trimethylsilyleth-1-yl, 1-Hydroxy-2-methylprop-1-yl, 1-Hydroxy-2,2-dimethylprop-1-yl, 1-Hydroxy-2,2-dimethyl-but-1-yl, 1-Hydroxy-2-ethyl-2-methylbut-1-yl, 1-Hydroxy-2,2-diethylbut-1-yl, Phenylmethyl, 1-Hydroxy-1-phenylmethyl, 2-Pyridylmethyl oder 3-Pyridylmethyl bedeutet,
wobei 2-Pyridylmethyl oder 3-Pyridylmethyl substituiert sein können mit 0, 1, 2 oder 3 Substituenten unabhängig voneinander ausgewählt aus der Gruppe bestehend aus Hydroxy, Amino, Trifluormethyl, Methyl, Methoxy und Morpholinyl,
- R²: Wasserstoff bedeutet,
- R³: 1-Amino-3-methylbut-1-ylcarbonyl, 1-Amino-3,3-dimethylbut-1-ylcarbonyl oder 1-Amino-2-trimethylsilyleth-1-ylcarbonyl bedeutet,
- R⁴: Wasserstoff bedeutet,
- R⁵: eine Gruppe der Formel 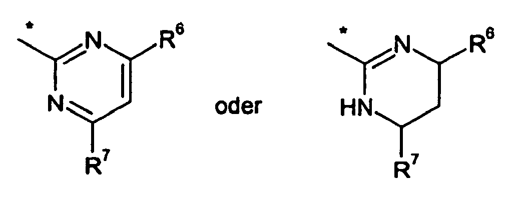 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
und ihre Salze, ihre Solvate und die Solvate ihrer Salze.

Bevorzugt sind auch Verbindungen der Formel (I), in welcher
- R¹: 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
- R²: Wasserstoffbedeutet,
- R³: 1-Amino-3,3-dimethylbut-1-ylcarbonyl oder 1-Amino-2-trimethylsilyleth-1-ylcarbonyl bedeutet,
- R⁴: Wasserstoff bedeutet,
- R⁵: eine Gruppe der Formel 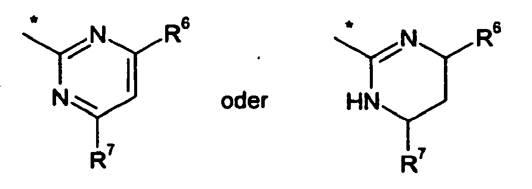 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
und ihre Salze, ihre Solvate und die Solvate ihrer Salze.

Bevorzugt sind auch Verbindungen der Formel (I), in welcher
- R¹: 2-Methylprop-1-yl bedeutet,
- R²: Wasserstoff bedeutet,
- R³: 1-Amino-3-methylbut-1-ylcarbonyl bedeutet,
- R⁴: Wasserstoff bedeutet,
- R⁵: eine Gruppe der Formel 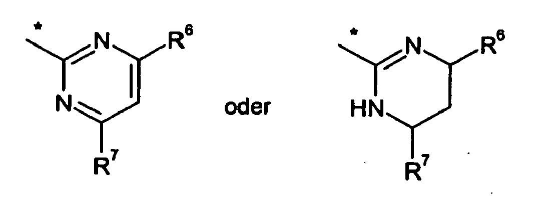 bedeutet,
wobei
* die Anknüpfstelle an das Stickstoffatom bedeutet,
R⁶ und R⁷ unabhängig voneinander C₁-C₆-Alkyl bedeuten,
und ihre Salze, ihre Solvate und die Solvate ihrer Salze.

Bevorzugt sind auch Verbindungen der Formel (I), in welcher
- R¹: 2,2-Dimethylprop-1-yl bedeutet,
- R²: Wasserstoff bedeutet,
- R³: 1-Amino-3,3-dimethylbut-1-ylcarbonyl bedeutet,
- R⁴: Wasserstoff bedeutet,
- R⁵: eine Gruppe der Formel 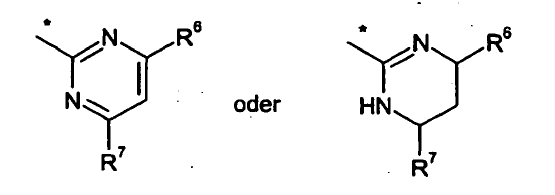 bedeutet,
wobei
* die Anknüpfstelle an das Stickstoffatom bedeutet,
R⁶ und R⁷ unabhängig voneinander C₁-C₆-Alkyl bedeuten,
und ihre Salze, ihre Solvate und die Solvate ihrer Salze.

Bevorzugt sind auch Verbindungen der Formel (I), in welcher
- R¹: 2-Methylprop-1-yl, 2,2-Dimethylprop-1-yl, 2,2-Dimethylbut-1-yl, 2-Trimethylsilyleth-1-yl, 1-Hydroxy-2-methylprop-1-yl, 1-Hydroxy-2,2-dimethylprop-1-yl, 1-Hydroxy-2,2-dimethylbut-1-yl, 1-Hydroxy-2-ethyl-2-methylbut-1-yl, 1-Hydroxy-2,2-diethylbut-1-yl, Phenylmethyl, 1-Hydroxy-1-phenylmethyl, 2-Pyridylmethyl oder 3-Pyridylmethyl bedeutet,
wobei 2-Pyridylmethyl oder 3-Pyridylmethyl substituiert sein können mit 0, 1, 2 oder 3 Substituenten unabhängig voneinander ausgewählt aus der Gruppe bestehend aus Hydroxy, Amino, Trifluormethyl, Methyl, Methoxy und Morpholinyl.

Bevorzugt sind auch Verbindungen der Formel (I), in welcher das von einer Aminosäure stammende Stereozentrum in R³ D-Konfiguration hat.

Bevorzugt sind auch Verbindungen der Formel (I), in welcher R¹ 2,2-Dimethylprop-1-yl und R² Wasserstoff bedeutet.

Bevorzugt sind auch Verbindungen der Formel (I), in welcher R³ 1-Amino-3,3-dimethylbut-1-ylcarbonyl und R⁴ Wasserstoff bedeutet.

Die in den jeweiligen Kombinationen bzw. bevorzugten Kombinationen von Resten im einzelne angegebenen Restedefinitionen werden unabhängig von den jeweiligen angegebenen Kombinationen der Reste beliebig auch durch Restedefinitionen anderer Kombination ersetzt.

Ganz besonders bevorzugt sind auch Kombinationen von zwei oder mehreren der oben genannten Vorzugsbereiche.

Gegenstand der Erfindung ist weiterhin ein Verfahren zur Herstellung der Verbindungen der Formeln (Ic), wobei nach Verfahren
[A] Verbindungen der Formel 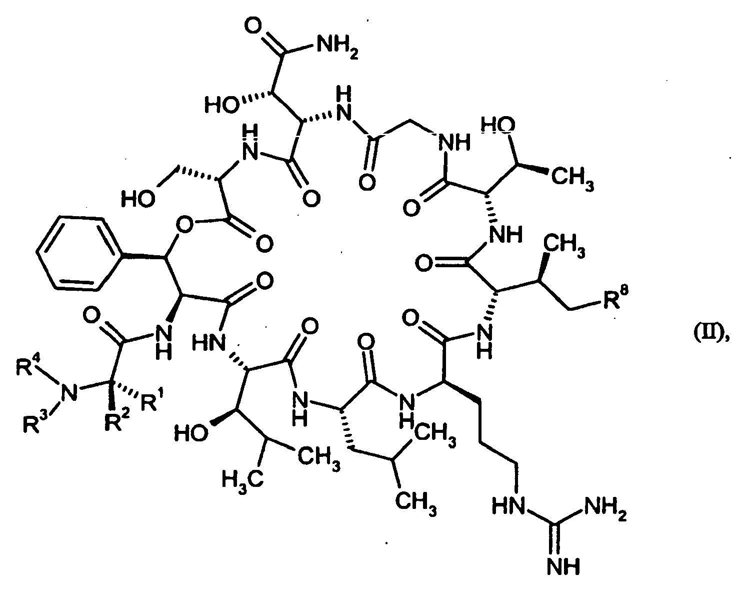 in welcher R¹, R², R³, R⁴ und R⁸ die oben angegebene Bedeutung haben,
   mit Verbindungen der Formel 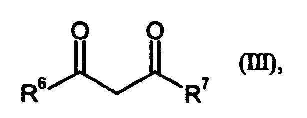 in welcher R⁶ und R⁷ die oben angegebene Bedeutung haben,
   zu Verbindungen der Formel 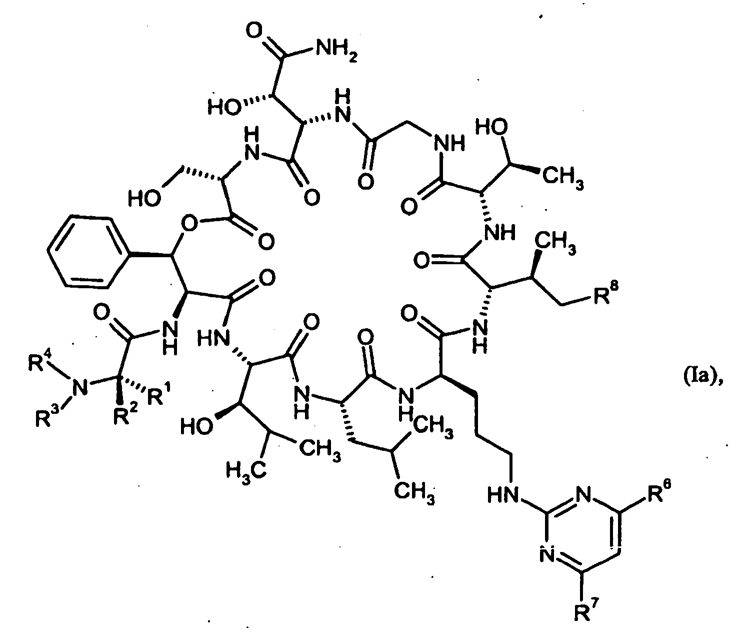 in welcher R¹, R², R³, R⁴, R⁶, R⁷ und R⁸ die oben angegebene Bedeutung haben,
   oder
[B] Verbindungen der Formel (Ia) mit einem Reduktionsmittel zu Verbindungen der Formel 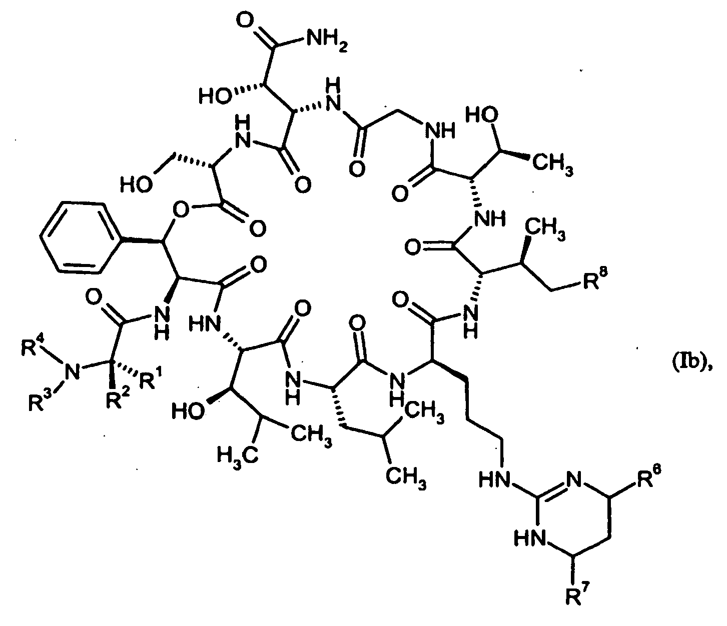 in welcher R¹, R², R³, R⁴, R⁶, R⁷ und R⁸ die oben angegebene Bedeutung haben,
umgesetzt werden.

Die Verbindungen der Formel (Ic) bestehen sich aus den Verbindungen der Formeln (Ia) und (Ib).

Freie Amino-Gruppen in den Resten R¹, R², R³ und R⁴ werden vor der Umsetzung gegebenenfalls nach dem Fachmann bekannten Methoden zum Beispiel mit einer Boc-Schutzgruppe oder einer Z-Schutzgruppe geschützt, die nach der Umsetzung wieder abgespalten wird.

Die Verbindungen der Formel (II) sind bekannt oder können hergestellt werden, indem die Verbindung der Formel 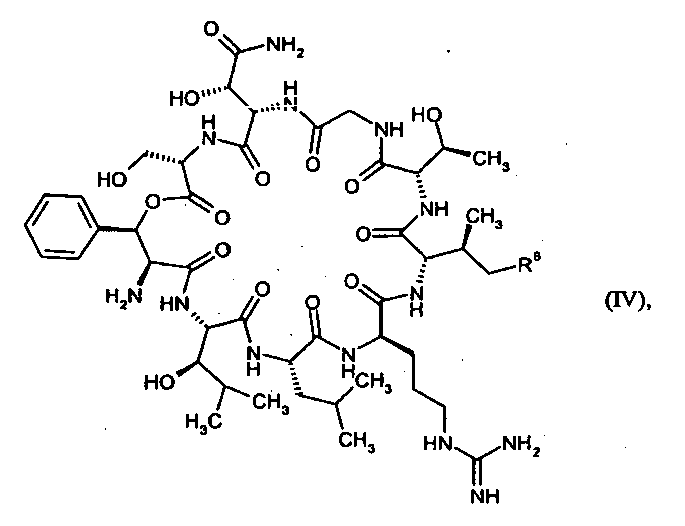 in welcher
R⁸ die oben angegebene Bedeutung hat,
mit Verbindungen der Formel 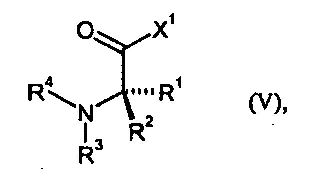 in welcher
- R¹, R², R³ und R⁴: die oben angegebene Bedeutung haben, und
- X¹: Halogen, bevorzugt Brom, Chlor oder Fluor, oder Hydroxy bedeutet,
umgesetzt wird.

Falls X¹ für Halogen steht, erfolgt die Umsetzung im Allgemeinen in inerten Lösungsmitteln, gegebenenfalls in Gegenwart einer Base, bevorzugt in einem Temperaturbereich von -30°C bis 150°C bei Normaldruck.

Inerte Lösungsmittel sind beispielsweise Tetrahydrofuran, Methylenchlorid, Acetonitril, Pyridin, Dioxan oder Dimethylformamid. Als inerte Lösungsmittel sind Tetrahydrofuran oder Methylenchlorid bevorzugt.

Basen sind beispielsweise Triethylamin, Diisopropylethylamin oder *N*-Methylmorpholin, bevorzugt ist Diisopropylethylamin.

Falls X¹ für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N,N,'*-Dipropyl-, *N,N'*-Diisopropyl-, *N,N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N,N*'-tetra-methyluronium-hexafluorophosphat (HBTU), 2-(2-Oxo-1-(2H)-pyridyl)-1,1,3,3-tetramethyluroniumtetrafluoro-borat (TPTU) oder *O-*(7-Azabenzotriazol-1-yl)-*N,N,N',N'*-tetramethyl-uroniumhexafluorophophat (HATU), oder 1-Hydroxybenztriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder hydrogencarbonat, oder organische Basen wie Trialkylamine z.B. Triethylamin, *N*-Methylmorpholin, *N-*Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (V) tragen gegebenenfalls Schutzgruppen, so dass sich in diesen Fällen der Umsetzung von Verbindungen der Formel (IV) mit Verbindungen der Formel (V) eine Abspaltung der Schutzgruppen mit zum Beispiel Trifluoressigsäure nach dem Fachmann bekannten Methoden anschließt.

Die Verbindung der Formel (IV) lässt sich durch doppelten Edman-Abbau aus Lysobactin (Beispiel 1A) synthetisieren.

Die Verbindungen der Formeln (III) und (V) sind bekannt oder lassen sich nach bekannten Verfahren aus den entsprechenden Edukten synthetisieren.

Die Herstellung der erfindungsgemäßen Verbindungen kann durch folgendes Syntheseschema verdeutlicht werden.

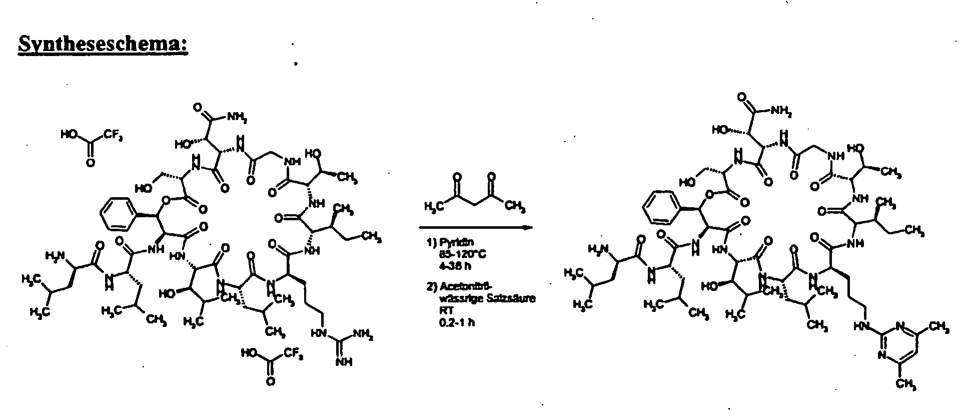

Die erfindungsgemäßen Verbindungen zeigen ein nicht vorhersehbares, wertvolles pharmakologisches Wirkspektrum. Sie zeigen eine antibakterielle Wirkung.

Sie eignen sich daher zur Verwendung zur Herstellung eines Arzneimittel zur Behandlung und/oder Prophylaxe von bakteriellen infektionen bei Menschen und Tieren.

Die erfindungsgemäßen Verbindungen zeichnen sich durch eine geringere Nephrotoxizität gegenüber Lysobactin aus.

Die beschriebenen Nonadepsipeptide wirken als Inhibitoren der bakteriellen Zellwandbiosynthese.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Grundsätzlich können die erfindungsgemäßen Zubereitungen gegen alle Bakterien und bakterienähnlichen Mikroorganismen verwendet werden, die im Besitz einer bakteriellen Zellwand (Murein sacculus) bzw. den dazugehörigen Enzymsystemen sind, beispielsweise durch die folgenden Erreger oder durch Mischungen der folgenden Erreger:
Gram-negative Kokken (*Neisseria gonorrhoeae*) sowie Gram-negative Stäbchen wie Enterobakteriaceen, z.B. *Escherichia coli, Hämophilus influenzae,* Pseudomonas, Klebsiella, Citrobacter (*C. freundii*, *C. divernis*), Salmonella und Shigella; ferner Enterobacter (*E. aerogenes, E. agglomerands*), Hafnia, Serratia (*S. marcescens*), Providencia, Yersinia, sowie die Gattung Acinetobacter, Branhamella und Chlamydia. Darüber hinaus umfasst das antibakterielle Spektrum strikt anaerobe Bakterien wie z.B. *Bacteroides fragilis*, Vertreter der Gattung Peptococcus, Peptostreptococcus sowie die Gattung Clostridium; ferner Mykobakterien, z.B. *M. tuberculosus*. Besonders ausgeprägte Wirkung zeigen die erfindungsgemäßen Verbindungen gegen Gram-positive Kokken, z.B. Staphylokokken (*S. aureus, S. epidermidis, S. haemolyticus, S. carnosus*), Enterokokken (*E. faecalis, E. faecium*) und Streptokokken (*S. agalactiae, S. pneumoniae, S. pyogenes*).

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:
Infektionskrankheiten beim Menschen wie z.B. unkomplizierte und komplizierte Harnwegsinfekte, unkomplizierte Haut- und Oberflächeninfektionen, komplizierte Haut- und Weichteilinfektionen, im Hospital und ambulant erworbene Lungenentzündung, nosokomiale Pneumonien, akute Exazerbationen und sekundäre bakterielle Infektionen der chronischen Bronchitis, akute Otitis media, akute Sinusitis, streptokokkale Pharyngitis, bakterielle Meningitis, unkomplizierte gonokokkale und nicht-gonokokkale Urethritis/Cervizitis, akute Prostatitis, Endocarditis, unkomplizierte und komplizierte intra-abdominale Infektionen, gynäkologische Infektionen, pelvische Entzündungskrankheit, bakterielle Vaginose, akute und chronische Osteomyelitis, akute bakterielle Arthritis, empirische Therapie in febrilen neutropenischen Patienten, desweiteren Bakterämien, MRSA Infektionen, akute infektiöse Diarrhoe, *Helicobacter pylori* Infektionen, postoperative Infektionen, odontogene Infektionen, ophthalmologische Infektionen, postoperative Infektionen (inkl. periproktaler Abszess, Wundinfektionen, Galleninfektionen, Mastitis und akute Appendizitis), zystische Fibrose und Bronchiectasis.

Außer beim Menschen können bakterielle Infektionen auch bei anderen Spezies behandelt werden. Beispielhaft seien genannt:
Schwein: Diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): *E. coli*-Infektionen, chronische Luflwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsia, Yersinia, erweitert.

Weiterer Gegenstand der vorliegenden Erfindung ist die Verwendung der erfindungsgemäßen Verbindungen zur Herstellung eines Arzneimittels zur Behandlung und/oder Prophylaxe von backteriellen Infektionen Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Bevorzugte Kombinationswirkstoffe sind antibakteriell wirkende Verbindungen, die ein anderes Wirkspektrum, insbesondere ergänzendes Wirkspektrum, haben und/oder synergistisch zu den erfindungsgemäßen Verbindungen sind.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzeiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginal kapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 100 mg/kg, vorzugsweise etwa 0.1 bis 10 mg/kg Körpergewicht zur Erzielung wirksamer Ergebnisse zu verabreichen, und bei oraler Applikation beträgt die Dosierung etwa 0.01 bis 50 mg/kg, vorzugsweise 0.5 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- Allg.: Allgemein(e)
- Area: (Peak)fläche
- ber.: berechnet
- BHI: Brain Heart Infusion
- Boc: *tert*-Butyloxycarbonyl
- br.: breites Signal (bei NMR-Spektren)
- Bsp.: Beispiel
- d: Dublett (bei NMR-Spektren)
- DC: Dünnschichtchromatographie
- DCI: direkte chemische Ionisation (bei MS)
- DCM: Dichlormethan
- DIEA: *N,N-*Diisopropylethylamin
- DMSO: Dimethylsulfoxid
- DMF: *N,N*-Dimethylformamid
- d. Th.: der Theorie
- EDC: 1-Ethyl-3-(3-dimethylaminopropyl)carbodiimide (auch EDCI)
- EDCxHCl: 1-Ethyl-3-(3-dimethylaminopropyl)carbodiimide-hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- gef.: gefunden
- ges.: gesättigt
- h: Stunde
- HATU: *O*-(7-Azabenzotriazol-1-yl)-*N,N,N',N'*-tetramethyluronium-hexafluorphosphat
- HOBt: 1-Hydroxybenzotriazol
- HPLC: Hochdruck-, Hochleistungsflüssigchromatographie
- HR: High Resolution (Hochauflösung)
- i. V.: im Vakuum
- konz.: konzentriert
- LC-MS: Flüssigchromatographie-gekoppelte Massenspektroskopie
- LDA: Lithium-Diisopropylamid
- m: middle (mittel) (bei UV- und IR-Spektren)
- m: Multiplett (bei NMR-Spektren)
- MALDI: Matrix-Assisted Laser Desorption/Ionization
- MHK: Minimale Hemmkonzentration
- min: Minute/Minuten
- MRSA: Methicillin-resistenter *Staphylococcus aureus*
- MS: Massenspektroskopie
- NCCLS: National Committee for Clinical Laboratory Standards
- neg.: negativ
- NMM: *N*-Methylmorpholin
- NMR: Kernresonanzspektroskopie (nuclear magnetic resonance)
- p.a.: pro analysi
- Pd-C: Palladium auf Kohle
- pos.: positiv
- proz.: Prozentig
- quant.: quantitativ
- RP-HPLC: Reverse Phase HPLC
- RT: Raumtemperatur
- Rₜ: Retentionszeit (bei HPLC)
- s: strong (stark) (bei UV- und IR-Spektren)
- s: Singulett (bei NMR-Spektren)
- TBTU: *O-*(Benzotriazol-1-yl)-*N,N,N',N*'-tetramethyluronium-Tetrafluoroborat
- TCTU: *O*-(1H-6-Chlorbenzotriazol-1-yl)-1,1,3,3-tetramethyluronium-Tetrafluoroborat
- TFA: Trifluoressigsäure
- TFE: 2,2,2-Trifluorethanol
- THF: Tetrahydrofuran
- TOF: Flugzeit (time of flight)
- UV: Ultraviolett
- Vis: sichtbar (visible)
- VRSA: Vancomycin-resistenter *Staphylococcus aureus*
- w: weak (schwach) (bei UV- und IR-Spektren)
- Z, Cbz: Benzyloxycarbonyl

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical Journal 1984, 219, 345-373. Sowie zitierte Literatur.
3. Zur Nomenklatur von Nonadepsipeptid-Derivaten, die in den Aminosäure-Seitenketten derivatisiert sind, wird das IUPÄC-Prefix-System zur Adressierung der jeweiligen Derivatisierungsstelle verwendet (IUPAC, Nomenclature and Symbolism for Amino Acids and Peptides, Names and Symbols for Derivatives of Named Peptides, Section 3AA-22, Recommendations 1983-1992). So bezeichnet beispielsweise *N^{ω.6}*-Acetyl-lysobactin ein an der Aminosäure 6 (vom *N*-Terminus des Depsipeptids gerechnet, d.h. hier D-Arg) speziell am terminalen Stickstoffatom acetyliertes Lysobactin.

### Allgemeine Methoden LC-MS, HR-MS, HPLC und Gelchromatographie

**Methode 1 (HPLC):** Gerätetyp HPLC: HP 1100 Series; UV DAD Säule: Zorbax Eclipse XBD-C8 (Agilent), 150 mm x 4.6 mm, 5 µm; Eluent A: 5 ml HClO₄/l Wasser, Eluent B: Acetonitril; Gradient: 0-1 min 10%B, 1-4 min 10-90%B, 4-5 min 90%B; Fluss: 2.0 ml/min; Ofen: 30°C; UV-Detektion: 210 und 254 nm.
**Methode 2 (HPLC):** Säule: Kromasil RP-18, 60 mm x 2 mm, 3.5 µm; Eluent A: 5 ml HClO₄/l Wasser, Eluent B: Acetonitril; Gradient: 0 min 2%B, 0.5 min 2%B, 4.5 min 90%B, 9 min 90%B; Fluss: 0.75 ml/min; Ofen: 30°C; UV-Detektion: 210 nm.
**Methode 3 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 4 (HPLC):** Säule: Kromasil RP-18, 250 mm x 4 mm, 5 µm; Eluent A: 5 ml HClO₄/l Wasser, Eluent B: Acetonitril; Gradient: 0 min 5%B, 10 min 95%B; Fluss: 1 ml/min; Ofen: 40°C; UV-Detektion: 210 nm.
**Methode 5 (HPLC):** Säule: Kromasil RP-18, 250 mm x 4 mm, 5 µm; Eluent A: 2 ml HClO₄/l Wasser, Eluent B: Acetonitril; Isokratisch: 45%B, 55%A; Fluss: 1 ml/min; Ofen: 40°C; UV-Detektion: 210 nm.
**Methode 6 (Gelchromatographie an Sephadex LH-20):** Gelchromatographie wird ohne Druck an Sephadex LH-20 (Firma Pharmacia) durchgeführt. Es wird nach LTV-Aktivität (UV Detektor für 254 nm, Firma Knauer) fraktioniert (Fraktionssammler ISCO Foxy 200). Säulendimensionen: 32 x 7 cm (1000-100 µmol Maßstab); 30 x 4 cm (100-10 µmol Maßstab); 25 x 2 cm (10-1 µmol Maßstab).
**Methode 7 (präparative HPLC):** Gerät: Gilson Abimed HPLC; UV-Detektor 210 nm; binäres Pumpensystem; Säule: Reprosil ODS-3, 5 µm, 250 x 20 mm; Eluent A: 0.2% Trifluoressigsäure in Wasser, Eluent B: Acetonitril; Flussrate: 25 ml/min; Säulentemperatur 40°C; 0-12 min 35% B.
**Methode 8 (LC-MS):** Gerätetyp MS: Micromass ZQ; Gerätetyp HPLC: HP 1100 Series; UV DAD; Säule: Phenomenex Synergi 2 µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 9 (HPLC):** Gerätetyp HPLC: HP 1050 Series; UV DAD 1100 Series; Säule Symmetry-Prep^{™}C₁₈, Firma Waters, 50 x 2.1 mm, 3.5 µm; Eluent A: Wasser/0.05% Trifluoressigsäure, Eluent B: Acetonitril; Gradient: 0-9 min 0-100% B, 9-11 min 100% B, 11-12 min 100-0% B, anschließend Regeneration der Chromatographiesäule. Ofen: 40°C, Fluss: 0.4 ml/min, UV-Detektion: 210 nm.
**Methode 10 (FT-ICR-HR-MS):** Die Massenpräzisionsmessungen werden an einem hochauflösenden Apex II Fourier-Transform Ionen-Cyclotron-Resonanz Massenspektrometer (Bruker Daltonik GmbH, Bremen) durchgeführt, das mit einem 7 Tesla Magneten, einer externen Elektrospray-Ionenquelle und einem Unix-basierten XMASS-Datensystem ausgestattet ist. Die Massenauflösung beträgt ca. 40.000 (50%-Tal-Definition).
**Methode 11 (Präparative HPLC):** Gerät: Gilson Abimed HPLC; UV-Detektor 210 nm; binäres Pumpensystem; Säule: Kromasil C-18, 5 µm, 100 Å, 250 x 20 mm; Eluent A: 0.2% Trifluoressigsäure in Wasser, Eluent B: Acetonitril: Flussrate: 25 ml/min; 0 min 20% B, Rampe 0-15 min 80% B, Rampe, 15-15.1 min 20% B, 15.1-20 min 20% B. Für *N-*Butoxycarbonyl-geschützte Substanzen wird die Trifluoressigsäure im Laufmittel grundsätzlich durch 0.05% Essigsäure ersetzt.
**Methode 12 (LC-MS): Gerätetyp MS**: Micromass ZQ; Gerätetyp HPLC: Waters Alliance 2790; Säule: Grom-Sil 120 ODS-4 HE 50 x 2 mm, 3.0 µm; Eluent A: Wasser + 500 µl 50%ige Ameisensäure / 1; Eluent B: Acetonitril + 500 µl 50%ige Ameisensäure / 1; Gradient: 0.0 min 0% B→ 0.2 min 0% B → 2.9 min 70% B → 3.1 min 90% B → 4.5 min 90% B; Ofen: 45°C; Fluss: 0.8 ml/min; UV-Detektion: 210 nm.
**Methode 13 (TOF-HR-ESI-MS):** TOF-HR-ESI-MS Spektren werden mit einem Micromass-LCT mass spectrometer (capillary 3.2 KV, cone 42 V, Quelle: 120°C). Die Proben werden mit einer Spritzenpumpe injiziert (Harvard Apparatus). Leucin-Enkephalin wird als Standart verwendet.
**Methode 14 (präparative HPLC):** Gerät: Gilson Abimed HPLC; UV Detektor 210 nm; binäres Pumpensystem; Säule: Nucleodur C18 Gravity, Firma Macherey-Nagel, 5 µm; 250 × 40 mm; Fluss: 15-45 mL/min; Eluent A: Wasser/0.1% Trifluoressigsäure, Eluent B: Acetonitril; Gradient: 0-12 min 10% B, 12-20 min 10-35% B, 20-25 min 35-40% B, 25-35 min 40% B, 35-45 min 40-50% B, 45-50 min 50-60% B 100% B, 50-60 min 60-100% B, 60-75 min 100% B, anschließend Regeneration der Chromatographiesäule.
**Methode 15 (MALDI-MS):** Die *MALDI-MS*/*MS-Untersuchungen* werden auf einem 4700 Proteomics Analyzer (Applied Biosystems, Framingham, MA, USA) durchgeführt, der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert.
**Methode 16 (LC-MS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5% B → 2.0 min 40% B→ 4.5 min 90% B → 5.5 min 90% B; Ofen: 45°C; Fluss: 0.0 min 0.75 ml/min → 4.5 min 0.75 ml/min → 5.5 min 1.25 ml/min; UV-Detektion: 210 nm.
**Methode 17 (präparative HPLC):** Gerät: Gilson Abimed HPLC; UV Detektor 210 nm; binäres Pumpensystem; Säule: Nucleodur C₁₈ Gravity, Firma Macherey-Nagel, 5 µm; 250 x 21 mm; Fluss: 20 ml/min; Eluent A: Wasser/0.25-0.5% Essigsäure, Eluent B: Acetonitril; Gradient: 0-3 min 5% B, 3-30 min 5-100% B, 30-38 min 100% B, anschließend Regeneration der Chromatographiesäule.
**Methode 18 (NMR, Quantitative TFA-Analyse/Absolutgehalte):** Eine gelöste Fluor-haltige organische Substanz und eine Kalibriersubstanz (z.B. 1,4-Dibromtetrafluorbenzol) werden eingewogen, ein geeignetes Lösungsmittel wird zugegeben und anschließend wird ein ¹⁹F-NMR Spektrum von der Probe aufgenommen (376 MHz). Aus dem NMR-Spektrum werden die notwendigen Integrale der Prüfsubstanz und der Kalibriersubstanz ermittelt. Hieraus wird der Gehalt an Fluor (bzw. TFA) bestimmt.
**Methode 19 (MALDI-MS):** Die *MALDI-MS*/*MS-Untersuchungen* werden auf einem 4700 Proteomics Analyzer (Applied Biosystems, Framingham, MA, USA) durchgeführt; der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert.
**Methode 20 (FT-ICR-HR-MS):** Die Massenpräzisionsmessungen werden an einem hochauflösenden Apex II Fourier-Transform Ionen-Cyclotron-Resonanz Massenspektrometer (Bruker Daltonik GmbH, Bremen) durchgerührt, das mit einem 7 Tesla Magneten, einer externen Elektrospray-Ionenquelle und einem Unix-basierten XMASS-Datensystem ausgestattet ist. Die Massenauflösung beträgt ca. 40.000 (50%-Tal-Definition).
**Methode 21 (präparative HPLC):** Gerät: Gilson Abimed HPLC; UV-Detektor 210 nm; binäres Pumpensystem; Säule: Reprosil ODS-A, 5 µm, 250 x 20 mm; Eluent A: 0.2% Trifluoressigsδure in Wasser, Eluent B: Acetonitril; Flussrate: 25 ml/min; Säulentemperatur 40°C; 0-10 min 20% B, 10-15 min 80% B.

### Allgemeine Arbeitsvorschriften

### Allgemeine Arbeitsvorschrift 1 (Hydrolytische Probenvorbereitung für MALDI-MS)

Das zu öffnende Depsipeptid (z.B. Lysobactin, 0.05 µmol) wird in einem Microvial zunächst mit einem Borat-Salzsäure Puffer (Fa. Merck) pH 8 (250 µl) versetzt. Man lässt über Nacht stehen, versetzt mit Essigsäure (100 µl) und gefriertrocknet die Probe. Das Rohprodukt wird ohne weitere Reinigungsschritte mittels MALDI-MS-Sequenzierung untersucht.

### Allgemeine Arbeitsvorschrift 2 (Edman^{0,5 und 1,5})

Zu einer Lösung des *N*-terminal freien Peptids (0.3 mmol) in trockenem Pyridin (30 ml) wird unter Argonschutzgasatmosphäre Phenylisothiocyanat (50 mmol) getropft. Das Reaktionsgemisch wird bei 37°C solange (ca. 1 h) gerührt bis die analytische HPLC-Kontrolle (Methode 13) ausreichenden Umsatz anzeigt (>95%). Das Reaktionsgemisch wird unter Temperaturkontrolle (<40°C) im Vakuum eingeengt und dann lyophilisiert.

### Allgemeine Arbeitsvorschrift 3 (Edman^{1,0 und 2,0})

Unter Argonschutzgasatomosphäre wird der Peptid-Thioharnstoff (0.2 mmol) als Feststoff bei starkem Rühren mit trockener Trifluoressigsäure versetzt und dann bei 40°C solange gerührt (ca. 20 min) bis die analytische HPLC-Kontrolle ausreichenden Umsatz (>95%) anzeigt. Das Reaktionsgemisch wird zügig bei Raumtemperatur (Temperaturkontrolle) im Vakuum eingeengt. Um das Rohprodukt von weiterer Trifluoressigsäure zu befreien, wird das Rohprodukt in Dichlormethan aufgenommen und erneut im Vakuum von Lösungsmittel befreit. Dieser Vorgang wird mehrfach mit Toluol (zweimal) und mit Dichlormethan (zweimal) wiederholt. Abschließend wird das Rohprodukt lyophilisiert.

### Ausgangsverbindungen

### Beispiel 1A

D-Leucyl-*N*¹-{(3S,6S,12S,15S,18R,21S,245,27S,28*R*)-6-[(1*S*)-2-amino-1-hydroxy-2-oxoethyl]-18-(3-{[amino(imino)methyl]amino}propyl)-12-[(1S)-1-hydroxyethyl]-3-(hydroxymethyl)-24-[(*1R*)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat (Lysobactin)

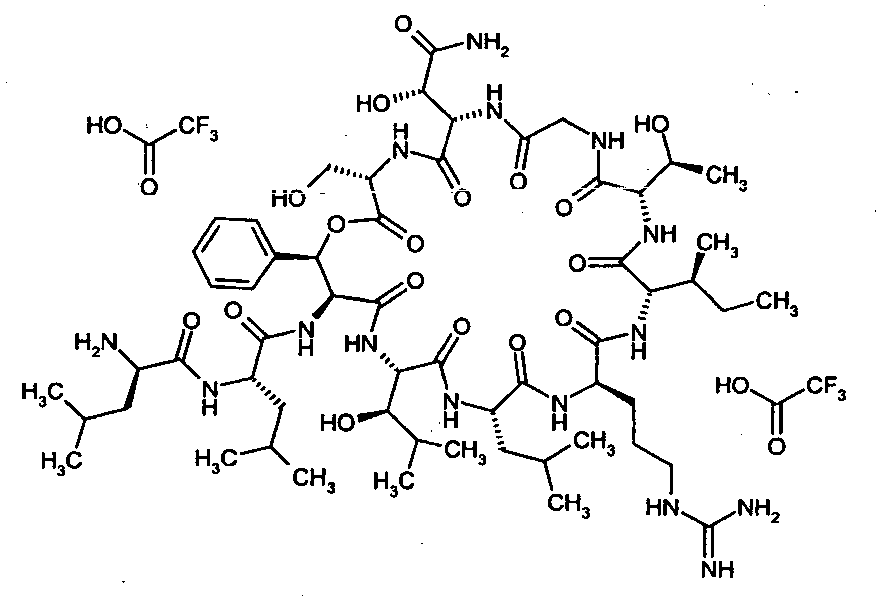

### Fermentation:

### Kultur Medium:

YM: Hefe-Malz Agar: D-Glucose (4 g/l), Hefe Extrakt (4 g/l), Malz Extrakt (10 g/l), 1 Liter Lewatit Wasser. Vor dem Sterilisieren (20 Minuten bei 121°C) wird der pH auf 7.2 eingestellt.

HPM: Mannit (5.4 g/l), Hefe Extrakt (5 g/l), Fleischpepton (3 g/l).

Arbeitskonserve: Der lyophilisierte Stamm (ATCC 53042) wird in 50 ml YM Medium angezogen.

Kolbenfermentation: 150 ml YM Medium oder 100 ml HPM Medium in einem 11 Erlenmeyerkolben werden mit 2 ml der Arbeitskonserve beimpft und für 30-48 Stunden bei 28°C auf einem Schüttler bei 240 Upm wachsen gelassen.

30 1 Fermentation: 300 ml der Kolbenfermentation (HPM) Medium) werden zum Animpfen einer sterilen 301 Nährmedienlösung verwandt (1 ml Antifoam SAG 5693/l). Diese Kultur wird für 21 Stunden bei 28°C, 300 Upm und einer Belüftung mit steriler Luft von 0.3 vvm wachsen gelassen. Der pH wird mit 1M Salzsäure auf pH = 7.2 konstant gehalten. Insgesamt werden während der Kultivierungszeit 880 ml 1M Salzsäure zugeführt.

Hauptkultur 2001): 15 x 150 ml YM Medium in 11 Erlenmeyerkolben werden mit 2 ml der Arbeitskonserve beimpft und bei 28°C für 48 Stunden und 240 Upm auf dem Schüttler wachsen gelassen. 2250 ml dieser Kultur werden zum Beimpfen einer sterilen 2001 Nährmedienlösung (YM) verwandt (1ml Antifoam SAG 5693/l) und für 18.5 Stunden bei 28°C, 150 Upm und einer Belüftung mit steriler Luft von 0.3 vvm wachsen gelassen.

Zur Kontrolle des Fermentationsverlaufs werden stündlich Proben (50 ml) entnommen. 2 ml dieser Kulturbrühe werden mit 1ml Methanol (0.5% Trifluoressigsäure) versetzt und über einen 0.45 µm Filter filtriert. 30 µl dieser Suspension werden mittels HPLC analysiert (Methode 1 und Methode 2).

Nach 18.5 Stunden wird die Kulturbrühe der Hauptkultur bei 17000 Upm in Überstand und Sediment getrennt.

### Isolierung:

Der Überstand (183 1) wird mit konzentrierter Trifluoressigsäure bzw. Natronlauge auf pH 6.5-7 eingestellt und auf eine Lewapolsäule (OC 1064, 60 l Inhalt) aufgetragen. Anschließend wird mit reinem Wasser, Wasser/Methanol 1:1 und anschließend mit reinem Methanol (mit 0.1% Trifluoressigsäure) eluiert. Diese organische Phase wird im Vakuum bis zu einem verbleibenden wässrigen Rest von 11.51 eingeengt.

Die verbleibende wässrige Phase wird an Kieselgel C₁₈ gebunden und aufgetrennt (MPLC, Biotage Flash 75, 75 x 30 cm, KP-C18-WP, 15-20 µm, Fluss: 30 ml; Eluent: Acetonitril/ Wasser mit 0.1% Trifluoressigsäure; Gradient: 10%, 15% and 40% Acetonitril). Die 40% Acetonitril Phase, die die Hauptmenge von Beispiel 1A enthält, wird im Vakuum eingeengt und anschließend lyophilisiert (∼13g). Dieses Feststoffgemisch wird in 1.2 g Portionen zunächst an einer präparativen HPLC (Methode 3), anschließend durch Gelfiltration an Sephadex LH-20 (5 x 70 cm, Acetonitril/Wasser 1:1, jeweils mit 0.05% Trifluoressigsäure) und einer weiteren präparativen HPLC (Methode 4) getrennt.

Dieser Prozess liefert 2250 mg Beispiel 1A.

Das Sediment wird in 41 Aceton/Wasser 4:1 aufgenommen, mit 2 kg Celite versetzt, mit Trifluoressigsäure auf pH = 6 eingestellt, ausgerührt und zentrifugiert. Das Lösungsmittel wird im Vakuum eingeengt und der Rückstand gefriergetrocknet. Das erhaltene Lyophilisat (89.9 g) wird in Methanol aufgenommen, abfiltriert, eingeengt und an Kieselgel (Methode 5) getrennt. Beispiel 1A wird danach per Gelfiltration (Sephadex LH-20, 5 x 68 cm, Wasser/Acetonitril 9:1 (mit 0.05% Trifluoressigsäure), Fluss: 2.7 ml/min. Fraktionsgröße 13.5 ml) zur Reinsubstanz aufgereinigt.

Dieser Prozess liefert 447 mg Beispiel 1A.

HPLC (Methode 1): Rₜ = 6.19 min

MS (ESIpos): m/z = 1277 (M+H)⁺

¹H NMR (500.13 MHz, *d₆*-DMSO): δ = 0.75 (d, 3H), 0.78 (d, 6H), 0.80 (t, 3H), 0.82 (d, 3H), 0.90 (d, 3H), 0.91 (d, 3H), 0.92 (d, 3H), 0.95 (d, 3H), 0.96 (d, 3H), 1.05 (m, 1H), 1.19 (d, 3H), 1.25 (m, 2H), 1.50 (m, 4H), 1.51 (m, 2H), 1.55 (m, 1H), 1.61 (m, 1H), 1.65 (m, 1H), 1.84 (m, 1H), 1.85 (m, 1H), 1.86 (m, 1H), 1.89 (m, 1H), 1.95 (m, 1H), 2.75 (m, 2H), 3.40 (m, 1H), 3.52 (m, 2H), 3.53 (dd, 1H), 3.64 (m, 2H), 3.66 (m, 1H), 3.68 (dd, 1H), 3.73 (m, 2H), 4.00 (dd, 1H), 4.02 (br., 1H), 4.13 (br., 1H), 4.32 (dd, 1H), 4.39 (t, 1H), 4.55 (m, 1H), 4.75 (dd, 1H), 5.19 (t, 1H), 5.29 (d, 1H), 5.30 (br., 1H), 5.58 (m, 2H), 6.68 (m, 3H), 6.89 (d, 1H), 6.93 (m, 3H), 6.94 (br., 1H), 6.98 (d, 1H), 7.12 (br., 1H), 7.20 (br., 2H), 7.23 (m, 2H), 7.42 (m, 2H), 7.54 (d, 1H), 7.58 (d, 1H), 8.32 (br., 1H), 9.18 (br., 1H), 9.20 (m, 2H), 9.50 (br., 1H).

¹³C-NMR (125.77 MHz, *d₆*-DMSO): δ = 10.3, 15.3, 19.0, 19.2, 19.6, 20.0, 20.9, 22.0, 22.4, 23.0, 23.2, 24.3, 24.4, 25.0, 25.4, 26.0, 27.8, 30.9, 35.4, 39.5, 40.8, 40.9, 41.6, 44.1, 51.5, 52.7, 55.9, 56.2, 56.4, 57.9, 58.8, 60.2, 61.1, 62.6, 70.1, 71.6, 71.7, 75.5, 128.1, 128.6, 136.7, 156.8, 168.2, 170.1, 170.4, 171.2, 171.5, 171.9, 172.2, 172.4, 173.7.

Die Zuordnung der Signale erfolgte nach der in der Literatur beschriebenen Zuordnung (T. Kato, H. Hinoo, Y. Terui, *J. Antibiot.,* **1988**, *61*, 719-725).

### Beisipiel 2A

### N^{2.1}-[1-Methyl-3-oxobut-1-en-1-yl]-N^{7.ω},N^{7.ω'}-(pent[2]en[2]yl[4]yliden)-lysobactin

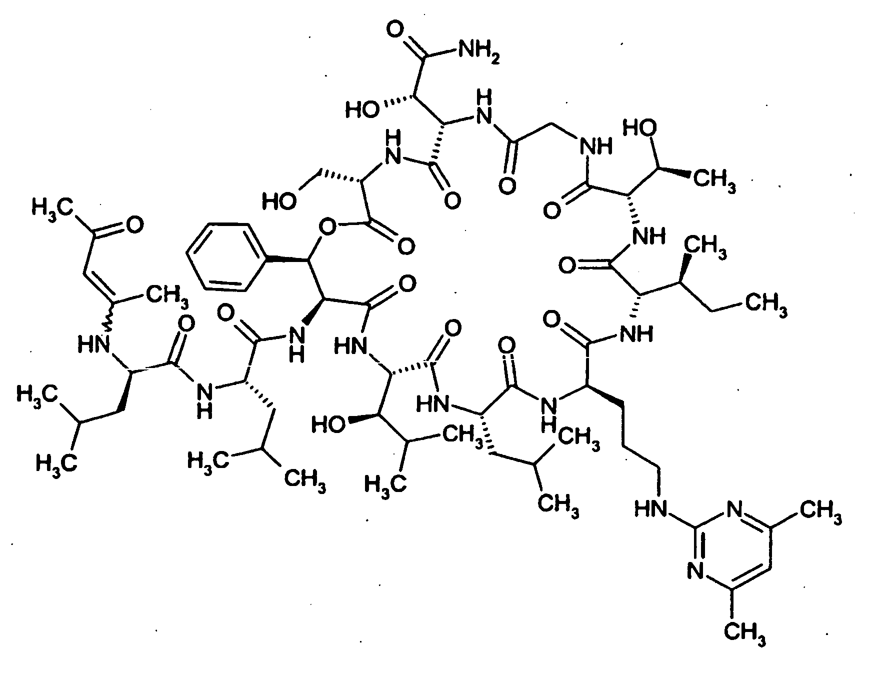

In einem druckfesten Reaktionsgefäß (Größe: 1 ml) werden zu einer Lösung von Lysobactin-bistrifluoracetat (15 mg, 0.01 mmol) in Pyridin (0.4 ml) gepulvertes Molsieb (4 Angström, 10 mg) und 2,4-Pentandion (200 Äquivalente, 0.2 ml, 2.0 mmol) gegeben. Das Reaktionsgemisch wird zunächst für 4 h auf 80°C und dann so lange auf 90°C erwärmt bis das HPLC-Chromatogramm vollständigen Umsatz anzeigt (ca. 12 h). Das Reaktionsgemisch wird noch heiß über eine Glasfritte filtriert (Porengröße 2), im Vakuum eingedampft und im Hochvakuum getrocknet (12 h). Der Rückstand wird mittels präparativer HPLC gereinigt (beispielsweise Methode 17 ohne TFA). Als Produkt erhält man einen Feststoff (8 mg, 54% d. Th.).

LC-MS (Methode 16): Rₜ = 3.43 min;

MS (ESIpos.): *m*/*z* (%) = 712 (100) [M + 2H]²⁺.

MS (ESIneg.): *m*/*z* (%) = 710 (100) [M - 2H]²⁻.

### Beispiel 3A

### N-Anilinocarbonothioyl)-D-leucyl-N¹-(3S,16S,12S,18R,21S,24S,27S,28R)-6-[(1S)-2-amino-1-hydroxy-2-oxoethyl]-18-(3-{[amino(imino)methyl]amino}propyl)12-[(1S)-1-hydroxyethyl]-3-(hydroxymethyl)-24-[(1R)-1-hydroxy-2-methylpropyl]-21-isobutl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### {N-(Anilinocarbonothioyl)lysobactin-monotrifluoracetat}

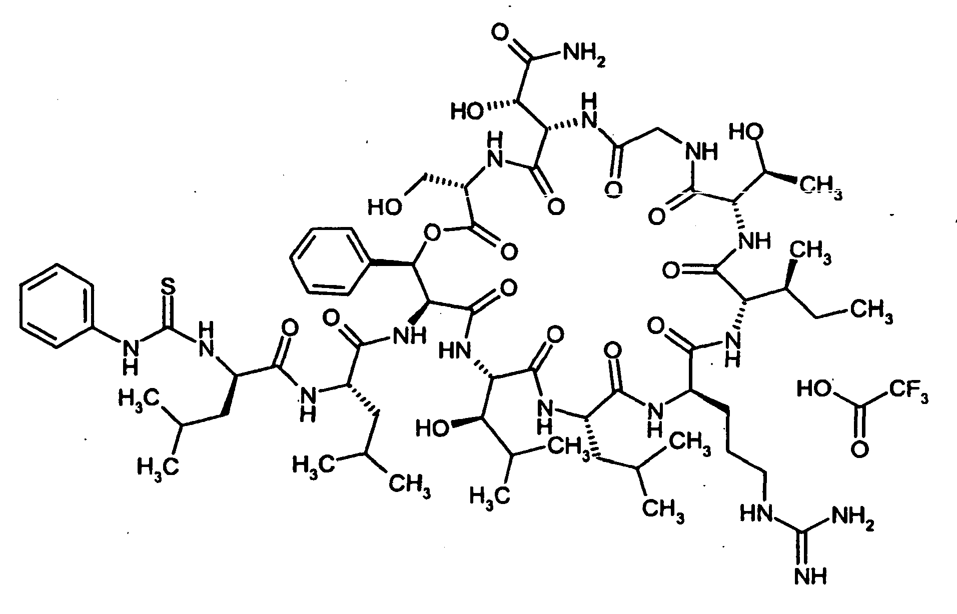

Gemäß der Allgemeinen Arbeitsvorschrift 2 wird Lysobactin-bistrifluoracetat (500 mg, 0.33 mmol) (Beispiel 1A) umgesetzt. Man erhält 600 mg (quant.) Produkt, das ungereinigt weiter umgesetzt werden kann.

Zur weiteren Aufreinigung kann das Rohprodukt gelchromatographiert (Methode 6; Methanol/0.1% Essigsäure) werden. Die Produkt-haltigen Fraktionen werden im Vakuum bei Raumtemperatur eingeengt und dann lyophilisiert. Man erhält das Produkt in 80% Ausbeute.

HPLC/UV-Vis (Methode 13): Rₜ = 6.84 min,

λₘₐₓ (qualitativ) = 220 nm (s), 248 (m), 269 (m).

LC-MS (Methode 11): Rₜ = 2.64 min;

MS (ESIpos.): m/z (%) = 706.5 (50) [M + 2H]²⁺, 1412 (20) [M + H]⁺;

LC-MS (Methode 12) : Rₜ = 4.95 min;

MS (ESIpos.): m/z (%) = 1412 (100) [M + H]⁺.

### Beispiel 4A

### N¹-{(3S,6S,12S,15S,18R,21S,24S,27S,28R)-6-[(1S)-2-Amino-1-hydroxy-2-oxoethyl]-18-(3-{[amino(imino)methyl]amino}propyl)-12-[(1S)-1-hydroxyethyl]-3-(hydroxy-methyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

### {Des-D-leucyllysobactin-bistrifluoracetat}

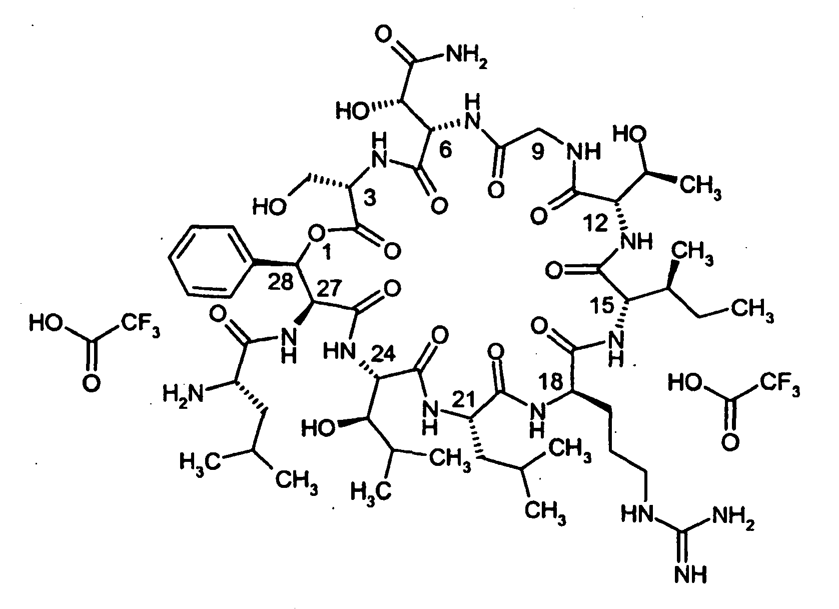

Thioharnstoff (Beispiel 3A) (300 mg, 0.2 mmol) wird gemäß der Allgemeinen Arbeitsvorschrift 3 umgesetzt. Das Rohprodukt wird gelchromatographiert (Methode 6; Methanol/0.25% Essigsäure) und anschließend mittels präparativer HPLC (Methode 8) feingereinigt. Man erhält 147 mg (65% d. Th.) Produkt.

HPLC/UV-Vis (Methode 13): Rₜ = 4.96 min,

λₘₐₓ (qualitativ) = 220 nm (s), 255-270 (w).

LC-MS (Methode 12): Rₜ = 3.84 min;

MS (ESIpos.): m/z (%) = 582.4 (100) [M + 2H]²⁺, 1164 (20) [M + H]⁺.

FT-ICR-HR-MS (Methode 20):

| | |
|---|---|
| C₅₂H₈₈N₁₄O₁₆ [M + 2H]²⁺ | ber. 582.32459, gef. 582.32460; |
| C₅₂H₈₇N₁₄NaO₁₆ [M + H + Na]²⁺ | ber. 593.31556, gef. 593.31564. |

Zur Aminosäure-Sequenzbestimmung wird eine analytische Probe des Produkts nach der Allgemeinen Arbeitsvorschrift 1 hydrolysiert.

MALDI-MS (Methode 19): m/z (%) = 1181.7 (100) [M + H]⁺.

### Alternatives Darstellungsverfahren in größerem Maßstab:

Beispiel 1A (6.47 g, 4.30 mmol) wird unter Argonatmosphäre in Pyridin (90 ml) gelöst. Dann wird Phenylisothiocyanat (1.16 g, 8.60 mmol, 2 Äquivalente) zugegeben und die Reaktionsmischung bei 37°C für 1 h gerührt. Anschließend wird das Lösungsmittel am Rotationsverdampfer abdestilliert und der Rückstand über Nacht am Ölpumpenvakuum getrocknet. Man erhält das Zwischenprodukt Beispiel 2A in einer Rohausbeute von 6.60 g. Das Zwischenprodukt wird ohne Reinigung weiter umgesetzt. Dazu wird Beispiel 3A (6.60 g) unter Argonatmosphäre in Trifluoressigsäure (107 ml) gelöst und 30 min bei Raumtemperatur gerührt. Dann wird die Lösung am Rotationsverdampfer unter Vakuum aufkonzentriert, kurz am Ölpumpenvakuum getrocknet, in Methyl-*tert*-butylether (250 ml) aufgenommen und so lange heftig gerührt, bis ein pulvriger amorpher Feststoff entstanden ist. Dieser wird mit Vakuum abfiltriert und mit Methyl-*tert*-butylether (200 ml) gewaschen, dann wird noch mit Dichlormethan (zweimal 100 ml) nachgewaschen. Der Feststoff wird in einen Kolben überführt und am Ölpumpenvakuum getrocknet. Man erhält Beispiel 4A in einer Rohausbeute von 6.0 g (quant.). Das Produkt kann ohne weitere Reinigung umgesetzt werden.

### Beispiel 5A

### N²-(Anilinocarbonothioyl)-N¹-{(3S,6S,12S,15S,18R,21S,24S,27S,28R)-6-[(1S)-2-amino-1-hydroxy-2-oxoethyl]-18-(3-{[amino(imino)methyl)amino}propyl)-12-[(1S)-1-hydroxyethyl)-3-(hydroxymethyl)-24-[(1R)-1-hydroxy-2-methylpropyl]-21-isobutyl-15-[(1S)-1-methylpropyl)-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

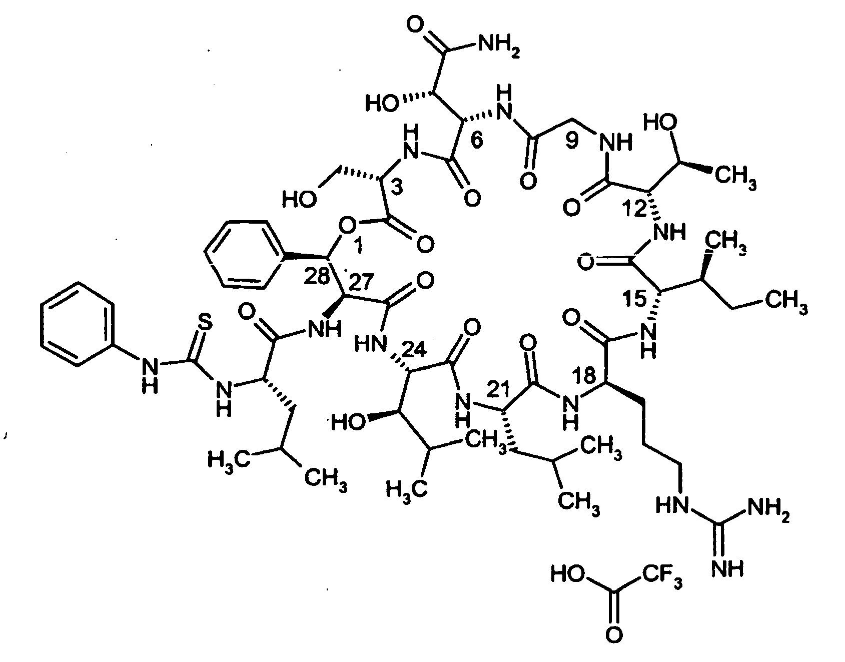

Gemäß der Allgemeinen Arbeitsvorschrift 2 wird Des-D-leucyllysobactin-bistrifluoracetat (Beispiel 4A, 255 mg, 0.18 mmol) umgesetzt. Man erhält 322 mg (quant.) Produkt, das ungereinigt weiter umgesetzt werden kann:

Zur weiteren Aufreinigung kann das Rohprodukt gelchromatographiert (Methode 6; Methanol/0.1% Essigsäure) werden. Die Produkt-haltigen Fraktionen werden im Vakuum bei Raumtemperatur eingeengt und dann lyophilisiert.

HPLC/UV-Vis (Methode 13): Rₜ = 6.56 min,
λₘₐₓ (qualitativ) = 220 nm (s), 245 (m), 268 (m).
LC-MS (Methode 12): Rₜ = 4.85 min;
MS (ESIpos.): m/z (%) = 1299 (100) [M + H]⁺.

### Beispiel 6A

### (2S)-2-{(3S,6S,12S,15S,18R,21S,24S,27S,28R)-27-Amino-18-(3-{[amino(imino)methyl]amino}-propyl)-12-[(1S-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-2 8-phenyl-1-oxa-4,7,10,13,16,19,22,25-octaazacyclooctacosan-6-yl}-2-hydroxyethanamid-bistrifluoracetat

### {Des(1-D-leucyl-2-L-leucyl)lysobactin-bistrifluoracetat}

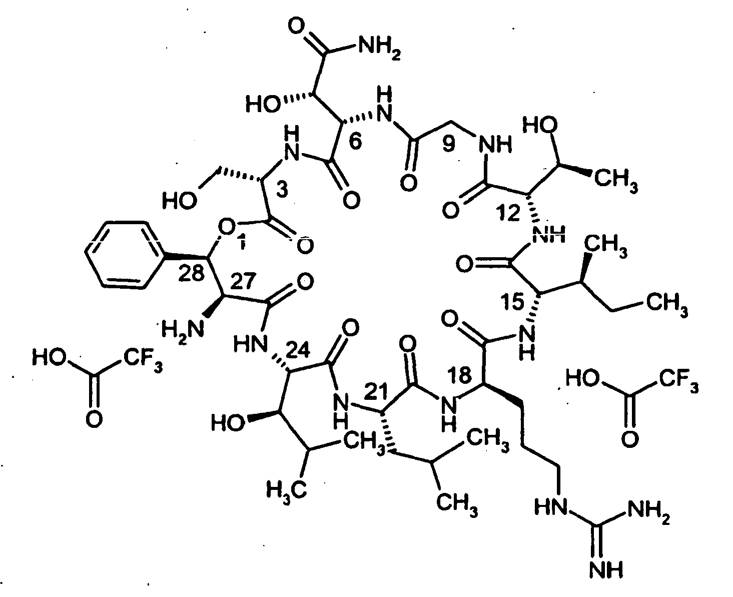

Der Thioharnstoff (Beispiel 5A, 66 mg, 34 µmol) wird gemäß der Allgemeinen Arbeitsvorschrift 3 umgesetzt. Das Rohprodukt kann durch zügige Gelchromatographie (Methode 6; Methanol/0.25% Essigsäure) vorgereinigt werden. Präparative HPLC (Methode 8 oder Methode 9 gefolgt von anschließendem Umsalzen des Chromatographieprodukts durch Zusatz von TFA (100 µmol)) liefert 45 mg (75% d. Th.) Produkt.

HPLC/UV-Vis (Methode 13): Rₜ = 4.71 min,

λₘₐₓ (qualitativ) = 220 nm (s), 255-270 (w).

LC-MS (Methode 11): Rₜ = 1.65 min;

MS (ESIpos.): m/z (%) = 526 (100) [M + 2H]²⁺, 1051 (15) [M + H]⁺.

### Alternatives Darstellungsverfahren in größerem Meßstab:

Beispielverbindung 4A (6.47 g, 4.30 mmol) wird unter Argonatmosphäre in Pyridin (92 ml) gelöst. Dann wird Phenylisothiocyanat (8.75 g, 64.68 mmol, 15 Äquivalente) zugegeben und die Reaktionsmischung bei 37°C für 1 Stunde gerührt. Anschließend wird das Lösungsmittel am Rotationsverdampfer abdestilliert und der Rückstand über Nacht am Ölpumpenvakuum getrocknet. Man erhält Beispiel 5A in einer Rohausbeute von 6.0 g. Das Zwischenprodukt wird ohne Reinigung weiter umgesetzt. Dazu wird das rohe Beispiel 5A unter Argonatmosphäre in Trifluoressigsäure (82 ml) gelöst und 30 min bei Raumtemperatur gerührt. Dann wird die Lösung am Rotationsverdampfer unter Vakuum aufkonzentriert, kurz am Ölpumpenvakuum getrocknet, in Methyl*-tert-*butylether (250 ml) aufgenommen und so lange heftig gerührt, bis ein pulvriger amorpher Feststoff entstanden ist. Dieser wird mit Vakuum abfiltriert und mit weiterem Methyl-*tert*-butylether (200 ml) gewaschen, dann wird noch mit zwei Portionen je 100 ml Dichlormethan nachgewaschen. Der Feststoff wird in einen Kolben überführt und am Ölpumpenvakuum getrocknet. Man erhält die Titelverbindung in einer Rohausbeute von 5.4 g (quant.). Das Produkt wird durch präparative HPLC weiter aufgereinigt (Methode 21). Man erhält 1.79 g der Titelverbindung (32% d. Th.).

### Ausführungsbeispiele

### Beispiele 1

### N^{ω.6},N^{ω'.6}-(Pent[2]en[2]yl[4]yliden)-lysobactin-trifluoracetat

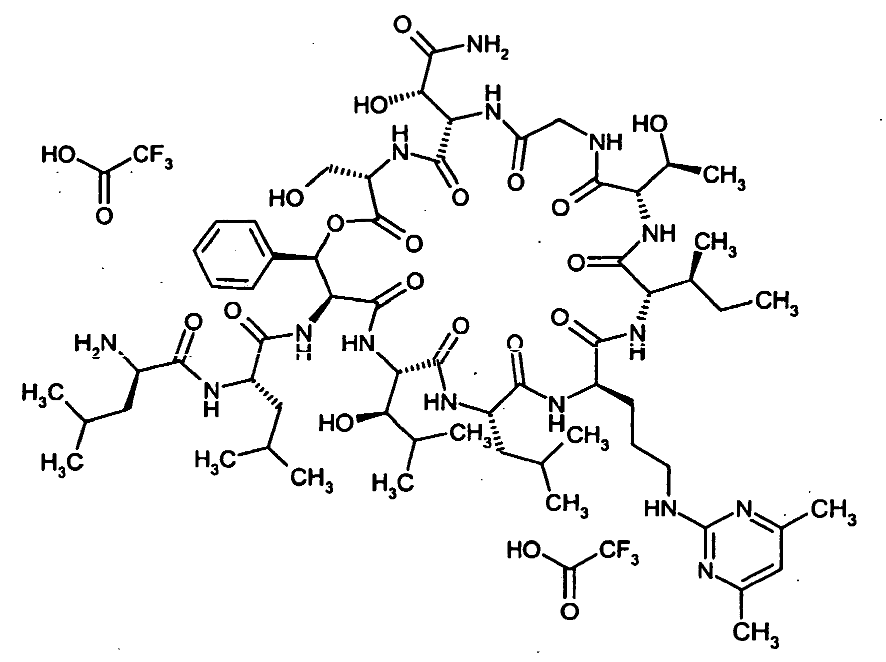

In einem mit Rückflusskühler ausgerüsteten Dreihalskolben werden zu einer Lösung von Lysobactin-bistrifluoracetat (2.0 g, 0.8 mmol) in Pyridin (55 ml) gepulvertes Molsieb (4 Angström, 0.5 g) und 2,4-Pentandion (40 Äquivalente, 3.3 ml, 32.1 mmol) gegeben. Das Reaktionsgemisch wird zunächst für 3.5 h auf 85°C und dann so lange auf 110°C erwärmt bis das HPLC-Chromatogramm vollständigen Umsatz anzeigt (ca. 4-8 h). Das Reaktionsgemisch wird noch heiß über eine Glasfritte filtriert (Porengröße 2), im Vakuum eingedampft und im Hochvakuum getrocknet (12 h). Der Rückstand (1.9 g) wird in einem Gemisch aus Acetonitril (30 ml) und 0.5 N wässriger Salzsäure (40 ml) aufgenommen und so lange bei Raumtemperatur gerührt, bis das HPLC-Chromatogramm vollständigen Umsatz anzeigt (ca. 0.4 h). Das Reaktionsgemisch wird im Vakuum eingeengt, eingefroren und gefriergetrocknet. Das Spaltungsprodukt wird mittels Gelchromatographie (Methode 6, Laufmittel Methanol/Essigsäure 99/1) aufgereinigt, wobei man 1.5 g Rohprodukt erhält, das anschließend mittels präparativer HPLC (Methode 7) feingereinigt wird. Man erhält 536 mg (46% d. Th.) Produkt.

EPLC/UV-Vis (Methode 9): Rₜ = 5.9 min,

λₘₐₓ (qualitativ) = 220 nm (s), 310 (s).

LC-MS (Methode 8): Rₜ = 1.45 min;

MS (ESIpos.): *m*/*z* (%) = 671 (100) [M + 2H]²⁺, 1341 (10) [M + H]⁺.

MS (ESIneg.): *m*/*z* (%) = 669 (80), 1339 (50) [M - H]⁻, 1385 [M - H + HCO₂H]⁻.

FT-ICR-HR-MS (Methode 10): C₆₃H₁₀₃N₁₅O₁₇ [M+ 2H]²⁺ ber 670.88227, gef. 670.88169

TOF-HR-ESI-MS (Methode 13): C₆₃H₁₀₂N₁₅O₁₇ [M + H]⁺ ber. 1340.7578, gef. 1340.7552;

Zur Aminosäure-Sequenzbestimmung wird eine analytische Probe des Produkts nach der Allgemeinen Arbeitsvorschrift 1 hydrolysiert.

MALDI-MS (Methode 15): *m*/*z* (%) = 1358.8 (100) [M + H]⁺.

Der TFA-Gehalt wird *via* ¹⁹F-NMR bestimmt (Methode 18; Kalibriersubstanz 1,4-Dibromtetrafluorbenzol): ber. 14.5 Gew% TFA, gef. 13.8 Gew% TFA.

### Beispiel 2

### N^{ω.6},N^{ω'.6}-(1,1,1,5,5,5-Hexafluor-pent[2]en[2]yl[4]yliden)-lysobactin

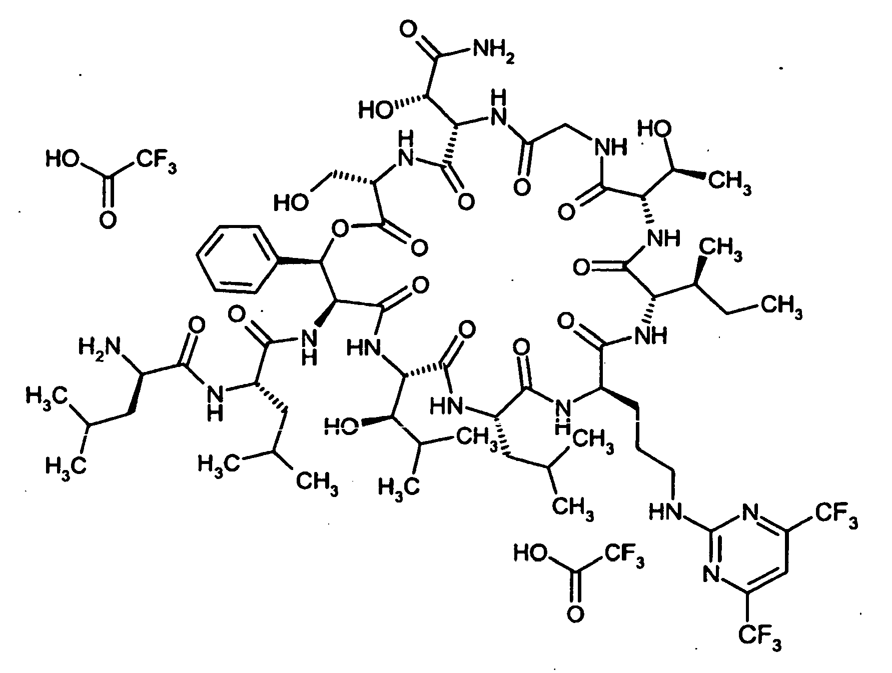

In einem mit Rückflusskühler ausgerüsteten Dreihalskolben werden zu einer Lösung von Lysobactin-bistrifluoracetat (10.0 mg, 0.05 mmol) in Pyridin (5 ml) gepulvertes Molsieb (4 Angström, 0.05 g) und 1,1,1,5,5,5-Hexafluor-2,4-pentandion (10 Äquivalente, 70 µl, 480 µmol) gegeben. Das Reaktionsgemisch wird zunächst für 48 h auf 85°C und dann so lange auf 95°C erwärmt bis das HPLC-Chromatogramm vollständigen Umsatz anzeigt (ca. 12 h). Das Reaktionsgemisch wird noch heiß über eine Glasfritte filtriert (Porengröße 2), im Vakuum eingedampft und im Hochvakuum getrocknet (12 h). Der Rückstand wird in einem Gemisch aus Acetonitril (3 ml) und 0.5 N wässriger Salzsäure (4 ml) aufgenommen und so lange bei Raumtemperatur gerührt, bis das HPLC-Chromatogramm vollständigen Umsatz anzeigt (ca. 0.5 h). Das Reaktionsgemisch wird im Vakuum eingeengt, eingefroren und gefriergetrocknet. Das Spaltungsprodukt wird mittels präparativer HPLC (Methode 11) aufgereinigt. Man erhält 3.5 mg (4.6% d. Th) Produkt.

LC-MS (Methode 12): Rₜ = 2.68 min;

MS (ESIpos.): m/z (%) = 725 (100) [M + 2H]²⁺, 1449 (20) [M + H]⁺.

MS (ESIneg.): m/z (%) = 687 (50), 1447 (100) [M - H]⁻, 1493 (15) [M - H + HCO₂H]⁻.

FT-ICR-HR-MS (Methode 10): C₆H₉₅F₆N₁₅O₁₇ [M + 2H]²⁺ ber. 724.85400, gef. 724.85427

### Beispiel 3

### N^{ω.6}-N^{ω'.6}-(Pentan[2,4]diyl)-lysobactin

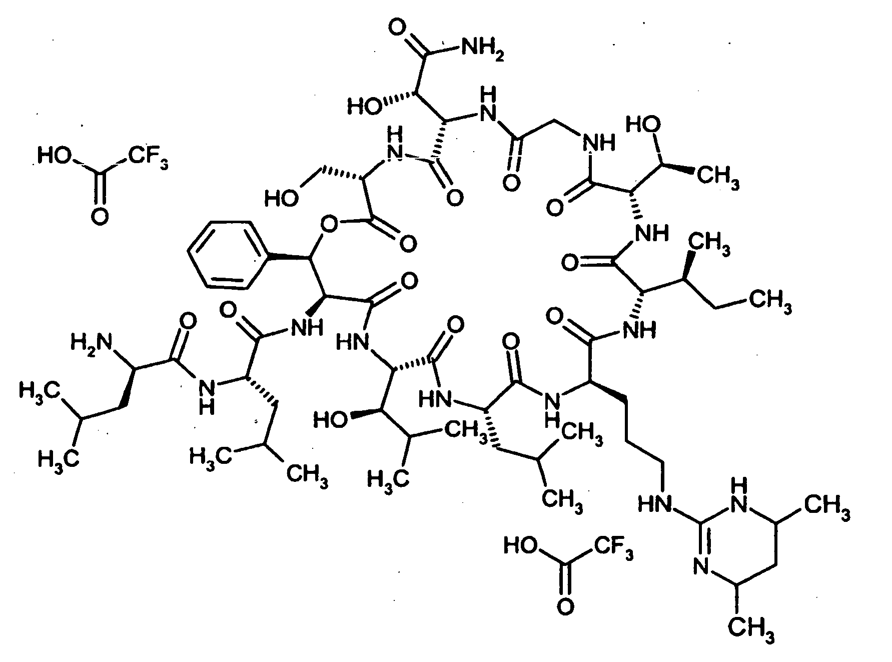

Eine Mischung von *N*^{ω.6},*N*^{ω'.6}-(Pent[2]en[2]yl[4]yliden)-lysobactin-trifluoracetat (205 mg, 0.14 mmol), 2-Propanol (10 ml), Wasser (10 ml), Palladium auf Kohle (10%, 100 mg) und konzentrierte Salzsäure (1.8 ml) wird bei Normaldruck und Raumtemperatur hydriert. Die Hydierung wird abgebrochen, wenn das HPLC-Chromatogramm vollständigen Umsatz anzeigt (ca. 24 h). Das Reaktionsgemisch wird über Celite filtriert (wobei mehrfach mit 2-Propanol nachgewaschen wird) und anschließend im Vakuum eingeengt. Das Rohprodukt wird mittels präparativer HPLC (Methode 14) gereinigt und gefriergetrocknet. Man erhält als Produkt einen Feststoff (52 mg, 25% d. Th.).

HPLC/UV-Vis (Methode 9): Rₜ = 6.0 min,

λₘₐₓ (qualitativ) = 220 nm (s), 260 (m).

LC-MS (Methode 8): Rₜ = 1.59 min;

MS (ESIpos.): m/z (%) = 673 (100) [M + 2H]²⁺, 1345 (10) [M + H]⁺.

MS (ESIneg.): m/z (%) = 671 (80) [M - 2H]²⁻, 1343 (40) [M - H]⁻, 1390 (100) [M - H + HCO₂H]⁻

TOF-HR-ESI-MS (Methode 13): C₆₃H₁₀₆N₁₅O₁₇ [M + H]⁺ ber. 1344.7891, gef. 1344.7867

### B. Bewertung der physiologischen Wirksamkeit

Die *in vitro*-Wirkung der erfindungsgemäßen Verbindungen kann in folgenden Assays gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staphylococcus aureus* 133, *Entercococcus faecalis* 27159, *E. faecium* 4147 und *Streptococcus pneumoniae* G9a werden mit den beschriebenen Testsubstanzen in einer 1:2 Verdünnungsreihe inkubiert. Die MHK-Bestimmung wird mit einer Zellzahl von 10⁵ Keimen pro ml in Isosensitest-Medium (Fa. Difco, Irvine/USA) durchgeführt, mit Ausnahme von *S*. *pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S*. *pneumoniae* in Gegenwart von 10% CO₂,

Die jeweils niedrigste Substanzkonzentration, bei der kein sichtbares Bakterienwachstum mehr auftritt, wird als MHK definiert. Die MHK-Werte werden in µg/ml angegeben.

Repräsentative in-vitro-Wirkdaten für die erfindungsgemäßen Verbindungen sind in Tabelle A wiedergegeben:

**Tabelle A**

| **Beispiel Nr.** | **MHK** ***S. aureus* 133** | **MHK** ***S. pneumoniae*** | **MHK** ***E. faecilis IGB 27159*** |
|---|---|---|---|
| 3 | 0.5 | 0.5 | 2 |

Die Eignung der erfindungsgemäßen Verbindungen zur Behandlung von bakteriellen Infektionen kann im folgenden Tiermodell gezeigt werden:

### Systemische Infektion mit Staphylococcus aureus 133:

Zellen von *S. aureus* 133 werden über Nacht in BHI-Bouillon (Fa. Oxoid, New York/ USA) angezüchtet. Die Übernachtkultur wird 1:100 in frische BHI-Bouillon verdünnt und für 3 Stunden inkubiert. Die dann in der logarithmischen Wachstumsphase befindlichen Zellen werden abzentrifugiert und zweimal mit gepufferter, physiologischer Kochsalzlösung gewaschen. Danach wird photometrisch eine Zellsuspension in Kochsalzlösung mit einer Extinktion von 50 Einheiten eingestellt. Nach einem Verdünnungsschritt (1:15) wird diese Suspension 1:1 mit einer 10%igen Mucinlösung gemischt. Von dieser Infektionslösung werden 0.25 ml/20 g Maus intraperitoneal (entsprechend 1x10⁶ Keimen/Maus) appliziert. Die Therapie erfolgt intraperitoneal oder intravenös 30 Minuten nach der Infektion. Für den Infektionsversuch werden weibliche CFW1-Mäuse verwendet. Das übergeben der Tiere wird über 6 Tage protokolliert.

Die Eigenschaften der erfindungsgemäßen Verbindungen im Hinblick auf die Nierenverträglichkeit können im folgenden Tiermodell gezeigt werden:

### Maus-Modell zur Bestimmung nephrotoxischer Effekte:

Nephrotoxische Nebenwirkungen der Nonadepsipeptide werden durch histopathologische Untersuchungen der Nieren in Mäusen und/oder Ratten nach mehrfacher Gabe einer bestimmten Dosierung analysiert. Dafür werden 5-6 Tiere täglich entweder intravenös (i.v.) oder intraperitoneal (i.p.) mit Substanzen behandelt, die in wässriger Lösung oder unter Zusatz von Solutol gelöst werden. Nierentoxische Effekte werden durch lichtmikroskopische Auswertung Hämatoxilin und Eosin (H&E) gefärbter Paraffinschnitte der Nieren bestimmt. Eine 'Periodic-Acid Schiff' (PAS)-Reaktion wird wahlweise zur besseren Darstellung von Glykoproteinen durchgeführt. Nephrotoxische Effekte werden semiquantitativ für jedes Tier als Schweregrade der auftretenden tubulären Basophilie und Degeneration/Regeneration festgelegt (Schweregrade: 0 = kein Effekt; 1 = minimaler Effekt; 2 = leichter Effekt; 3 = moderater Effekt; 4 = schwere Läsionen). Der durchschnittliche Schweregrad der tubulären Degeneration/Regeneration sowie die Inzidenz (Anzahl der betroffenen Tiere) wird pro Tiergruppe oder Derivat berechnet. Darüber hinausgehende Nierenveränderungen wie tubuläre Dilatation sowie Nekrosen und Ansammlung nekrotischen Materials werden ebenfalls aufgerührt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 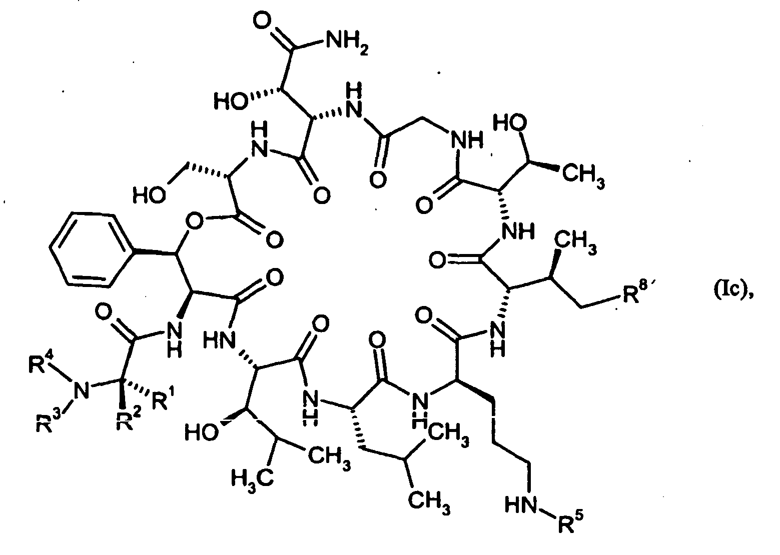 in welcher
R¹ Wasserstoff, C₁-C₆-Alkyl, C₂-C₆-Alkenyl, C₃-C₆-Cycloalkyl oder C₆-C₁₀-Aryl bedeutet,
wobei Alkyl, Alkenyl, Cycloalkyl und Aryl substituiert sein können mit 0, 1, 2 oder 3 Substituenten unabhängig voneinander ausgewählt aus der Gruppe bestehend aus Halogen, Hydroxy, Amino, Cyano, Trimethylsilyl, C₁-C₆-Alkyl, C₁-C₆-Alkoxy, Benzyloxy, C₃-C₆-Cycloalkyl, C₆-C₁₀-Aryl, 5- bis 7-gliedriges Heterocyclyl, 5- bis 10-gliedriges Heteroaryl, C₁-C₆-Alkylamino, C₆-C₁₀-Arylamino, C₁-C₆-Alkylcarbonylamino, C₆-C₁₀-Arylcarbonyl-amino, C₁-C₆-Alkylcarbonyl, C₁-C₆-Alkoxycarbonyl, C₆-C₁₀-Arylcarbonyl und Benzyloxy-carbonylamino,
worin Cycloalkyl, Aryl, Heterocyclyl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Trifluormethyl, C₁-C₆-Alkyl, C₁-C₆-Alkoxy, Phenyl und 5- bis 7-gliedriges Heterocyclyl,
R² Wasserstoff oder C₁-C₄-Alkyl bedeutet,
R³ C₁-C₆-Alkyl, C₃-C₆-Cycloalkyl, 5- bis 7-gliedriges Heterocyclyl, C₆-C₁₀-Aryl, 5- oder 6-gliedriges Heteroaryl, C₁-C₆-Alkylcarbonyl, C₁-C₆-Alkoxycarbonyl, C₃-C₆-Cycloalkylcarbonyl, 5- bis 7-gliedriges Heterocyclylcarbonyl, C₆-C₁₀-Arylcarbonyl, 5- oder 6-gliedriges Heteroarylcarbonyl oder C₁-C₆-Alkylanünocarbonyl bedeutet,
wobei Alkyl, Cycloalkyl, Heterocyclyl, Aryl, Heteroaryl, Alkoxycarbonyl; Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, C₁-C₆-Alkylamino und Phenyl,
und
wobei Alkylcarbonyl substituiert ist mit einem Substituenten Amino oder C₁-C₆-Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, C₁-C₆-Alkoxy, C₁-C₆-Alkylthio, Benzyloxy, C₃-C₆-Cycloalkyl, Phenyl, Naphthyl, 5- bis 10-gliedriges Heteroaryl, C₁-C₆-Alkylcarbonylamino, C₁-C₆-Alkoxy-carbonylamino, C₆-C₁₀-Arylcarbonylamino, C₆-C₁₀-Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, C₁-C₆-Alkyl, C₁-C₆-Alkoxy und Phenyl,
R⁴ Wasserstoff, C₁-C₄-Alkyl, Cyclopropyl oder Cyclopropylmethyl bedeutet,
R⁵ eine Gruppe der Formel 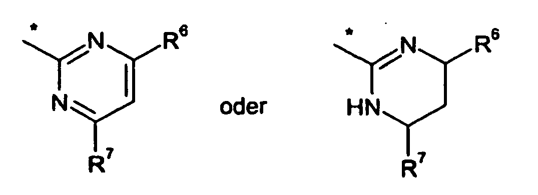 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
R⁸ Wasserstoff oder Methyl bedeutet,
oder eines ihrer Salze, ihrer Solvate oder der Solvate ihrer Salze.

2. Verbindung nach Anspruch 1, **dadurch gekennzeichnet, dass** sie der Formel 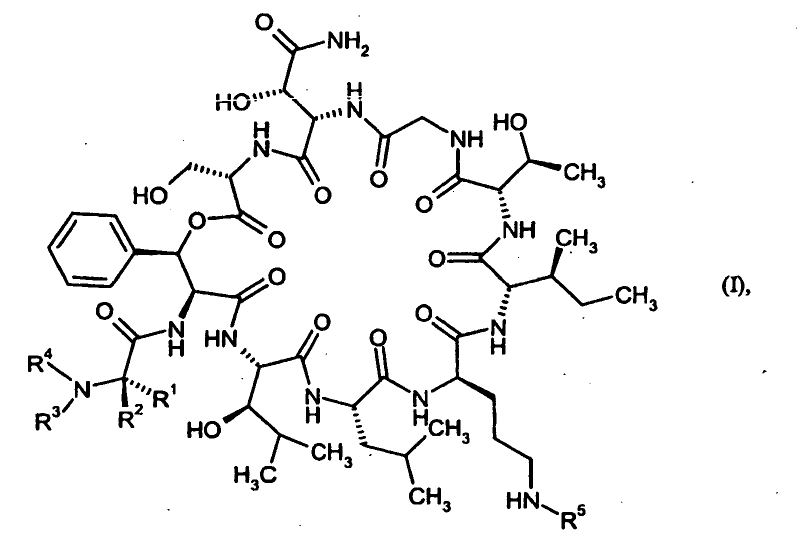 entspricht, in welcher
R¹ Wasserstoff, C₁-C₆-Alkyl, C₁-C₆-Alkenyl, C₃-C₆-Cycloalkyl oder C₆-C₁₀-Aryl bedeutet,
wobei Alkyl, Alkenyl, Cycloalkyl und Aryl substituiert sein können mit 0, 1, 2 oder 3 Substituenten unabhängig voneinander ausgewählt aus der Gruppe bestehend aus Halogen, Hydroxy, Amino, Cyano, Trimethylsilyl, C₁-C₆-Alkyl, C₁-C₆-Alkoxy, Benzyloxy, C₃-C₆-Cycloalkyl, C₆-C₁₀-Aryl, 5- bis 7-gliedriges Heterocyclyl, 5- bis 10-gliedriges Heteroaryl, C₁-C₆-Alkylamino, C₆-C₁₀-Arylamino, C₁-C₆-Alkylcarbonylamino, C₆-C₁₀-Arylcarbonyl-amino, C₁-C₆-Alkylcarbonyl, C₁₋C₆-Alkoxycarbonyl, C₆-C₁₀-Arylcarbonyl und Benzyloxy-carbonylamino,
worin Cycloalkyl, Aryl, Heterocyclyl und Heteroaryl ihrerseits substituiert sein können mit 0, 1, 2 oder 3 Substituenten unabhängig voneinander ausgewählt aus der Gruppe bestehend aus Halogen, Hydroxy, Amino, Cyano, Nitro, Trifluormethyl, C₁-C₆-Alkyl, C₁-C₆-Alkoxy, Phenyl und 5- bis 7-gliedriges Heterocyclyl,
R² Wasserstoff oder C₁-C₄-Alkyl bedeutet,
R³ C₁-C₆-Alkyl, C₃-C₆-Cycloalkyl, 5- bis 7-gliedriges Heterocyclyl, C₆-C₁₀-Aryl, 5- oder 6-gliedriges Heteroaryl, C₁-C₆-Alkylcarbonyl, C₁-C₆-Alkoxycarbonyl, C₃-C₆-Cycloalkylcarbonyl, 5- bis 7-gliedriges Heterocyclylcarbonyl, C₆-C₁₀-Aryl-carbonyl, 5- oder 6-gliedriges Hereroarylcarbonyl oder C₁-C₆-Alkylaminocarbonyl bedeutet,
wobei Alkyl, Cycloalkyl, Heterocyclyl, Aryl, Heteroaryl, Alkoxycarbonyl, Cycloalkylcarbonyl, Heterocyclylcarbonyl, Arylcarbonyl, Heteroarylcarbonyl und Alkylaminocarbonyl substituiert sein können mit 0, 1, 2 oder 3 Substituenten unabhängig voneinander ausgewählt aus der Gruppe bestehend aus Halogen, Hydroxy, Amino, C₁-C₆-Alkylamino und Phenyl,
und
wobei Alkylcarbonyl substituiert ist mit einem Substituenten Amino oder C₁-C₆-Alkylamino,
und
wobei Alkylcarbonyl substituiert sein kann mit weiteren 0, 1 oder 2 Substituenten unabhängig voneinander ausgewählt aus der Gruppe bestehend aus Halogen, Hydroxy, Trimethylsilyl, C₁-C₆-Alkoxy, C₁-C₆-Alkylthio, Benzyloxy, C₃-C₆-Cycloalkyl, Phenyl, Naphthyl, 5- bis 10-gliedriges Heteroaryl, C₁-C₆-Alkylcarbonylamino, C₁-C₆-Alkoxy-carbonylamino, C₆-C₁₀-Arylcarbonylamino, C₆-C₁₀-Arylcarbonyloxy, Benzyloxycarbonyl und Benzyloxycarbonylamino,
worin Phenyl und Heteroaryl ihrerseits substituiert sein können mit 0, 1, 2 oder 3 Substituenten unabhängig voneinander ausgewählt aus der Gruppe bestehend aus Halogen, Hydroxy, Nitro, C₁-C₆-Alkyl, C₁-C₆-Alkoxy und Phenyl,
R¹ Wasserstoff, C₁-C₄-Alkyl, Cyclopropyl oder Cyclopropylmethyl bedeutet,
R⁵ eine Gruppe der Formel 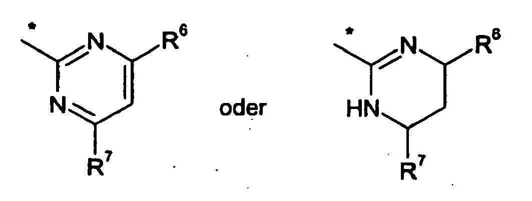 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
oder eines ihrer Salze, ihrer Solvate oder der Solvate ihrer Salze.

3. Verbindung nach Anspruch 2, **dadurch gekennzeichnet, dass**
R¹ 2-Methylprop-1-yl, 2,2-Dimethylprop-1-yl, 2,2-Dimethylbut-1-yl, 1-Trimethylsilylmethyl, 2-Trimethyl-silyleth-1-yl, 1-Hydroxy-2-methylprop-1-yl, 1-Hydroxy-2,2-dimethylprop-1-yl, 1-Hydroxy-2,2-dimethylbut-1-yl, 1-Hydroxy-2-ethyl-2-methylbut-1-yl, 1-Hydroxy-2,2-diethylbut-1-yl, Phenylmethyl, 1-Hydroxy-1-phenylmethyl, 2-Pyridylmethyl oder 3-Pyridylmethyl bedeutet,
wobei 2-Pyridylmethyl oder 3-Pyridylmethyl substituiert sein können mit 0, 1, 2 oder 3 Substituenten unabhängig voneinander ausgewählt aus der Gruppe bestehend aus Hydroxy, Amino, Trifluormethyl, Methyl, Methoxy und Morpholinyl,
R² Wasserstoff bedeutet,
R³ 1-Amino-3-methylbut-1-ylcarbonyl, 1-Amino-3,3-dimethylbut-1-ylcarbonyl oder 1-Amino-2-trimethylsilyleth-1-ylcarbonyl bedeutet,
R⁴ Wasserstoff bedeutet,
R⁵ eine Gruppe der Formel 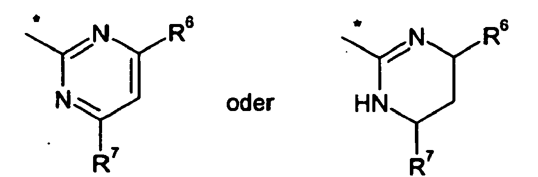 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
oder eines ihrer Salze, ihrer Solvate oder der Solvate ihrer Salze.

4. Verbindung nach einem der Ansprüche 2 oder 3, **dadurch gekennzeichnet, dass**
R¹ 2-Methylprop-1-yl bedeutet,
R² Wasserstoff bedeutet,
R³ 1-Amino-3-methylbut-1-ylcarbonyl bedeutet,
R⁴ Wasserstoff bedeutet,
R⁵ eine Gruppe der Formel 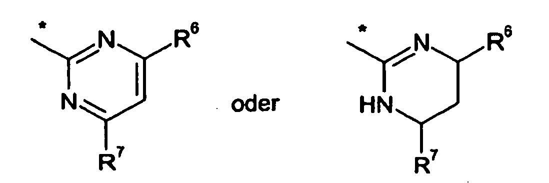 bedeutet,
wobei
* die Anknüpfstelle an das Stickstoffatom bedeutet,
R⁶ und R⁷ unabhängig voneinander C₁-C₆-Alkyl bedeuten,
oder eines ihrer Salze, ihrer Solvate oder der Solvate ihrer Salze.

5. Verbindung nach einem der Ansprüche 2 oder 3, **dadurch gekennzeichnet, dass**
R¹ 2,2-Dimethylprop-1-yl bedeutet, .
R² Wasserstoff bedeutet,
R³ 1-Amino-3,3-dimethylbut-1-ylcarbonyl bedeutet,
R⁴ Wasserstoff bedeutet,
R⁵ eine Gruppe der Formel 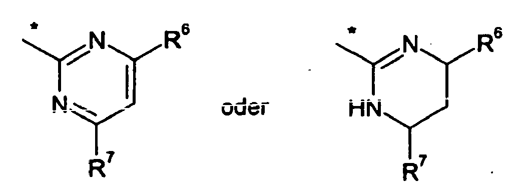 bedeutet,
wobei
* die Anknüpfstelle an das Stickstoffatom bedeutet,
R⁶ und R⁷ unabhängig voneinander C₁-C₆-Alkyl bedeuten,
oder eines ihrer Salze, ihrer Solvate oder der Solvate ihrer Salze.

6. Verbindung nach einem der Ansprüche 2 oder 3, **dadurch gekennzeichnet, dass**
R¹ 2,2-Dimethylprop-1-yl, 1-Trimethylsilylmethyl oder 3-Pyridylmethyl bedeutet,
wobei 3-Pyridylmethyl substituiert sein kann mit einem Substituenten Trifluormethyl,
R² Wasserstoff bedeutet,
R³ 1-Amino-3,3-dimethylbut-1-ylcarbonyl oder 1-Amino-2-trimethylsilyleth-1-ylcarbonyl bedeutet,
R⁴ Wasserstoff bedeutet,
R⁵ eine Gruppe der Formel 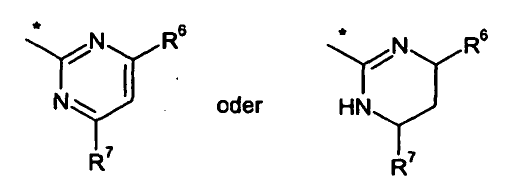 bedeutet,
wobei
* die Anknüpfstelle an das Stickstoffatom bedeutet,
R⁶ und R⁷ unabhängig voneinander C₁-C₆-Alkyl oder Trifluormethyl bedeuten,
oder eines ihrer Salze, ihrer Solvate oder der Solvate ihrer Salze.

7. Verfahren zur Herstellung einer Verbindung der Formel (Ic) nach Anspruch 1, **dadurch gekennzeichnet, dass**
nach Verfahren [A] eine Verbindung der Formel 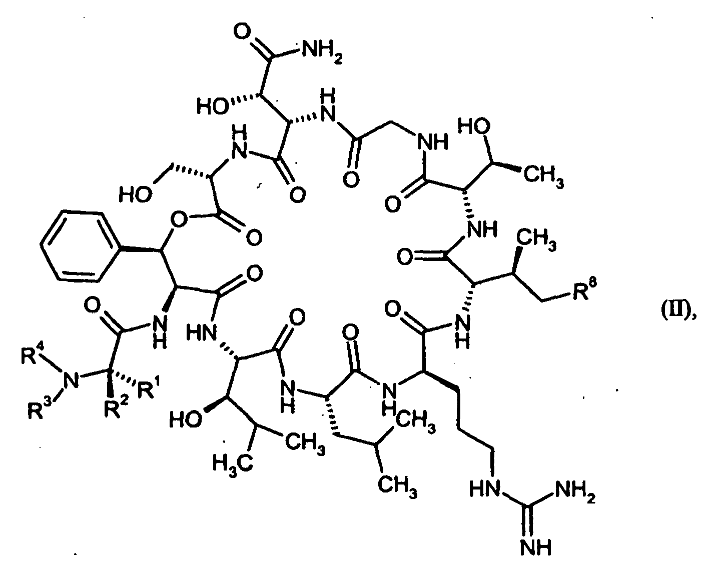 in welcher R¹, R², R³, R⁴ und R⁸ die in Anspruch 1 angegebene Bedeutung haben,
mit einer Verbindung der Formel 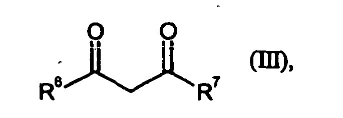 in welcher R⁶ und R⁷ die in Anspruch 1 angegebene Bedeutung haben,
zu einer Verbindung der Formel 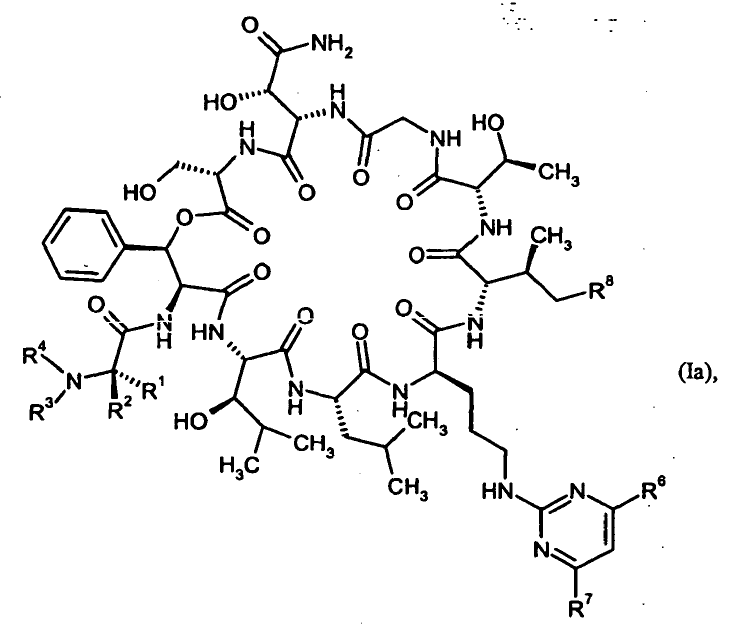 in welcher R¹, R², R³, W, R⁶, R⁷ und R⁸ die in Anspruch 1 angegebene Bedeutung haben,
oder
nach Verfahren [B] eine Verbindung der Formel (Ia) mit einem Reduktionsmittel zu einer Verbindung der Formel 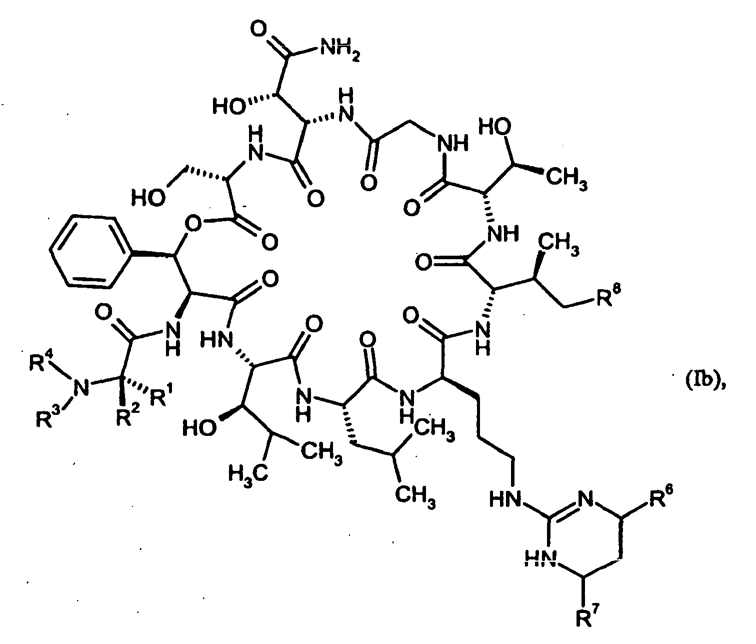 in welcher R¹, R², R³, R⁴, R⁶, R⁷ und R⁸ die in Anspruch 1 angegebene Bedeutung haben, umgesetzt wird.

8. Verbindung nach einem der Ansprüche 1 bis 6 zur Behandlung und/oder Prophylaxe von Krankheiten.

9. Verwendung einer Verbindung nach einem der Ansprüche 1 bis 6 zur Herstellung eines Arzneimittels zur Behandlung und/oder Prophylaxe von bakteriellen Infektionen.

10. Arzneimittel enthaltend eine Verbindung nach einem der Ansprüche 1 bis 6 in Kombination mit einem inerten, nichttoxischen, pharmazeutisch geeigneten Hilfsstoff.

11. Arzneimittel nach Anspruch 10 zur Behandlung und/oder Prophylaxe von bakteriellen Infektionen.

12. Verwendung einer antibakteriell wirksamen Menge mindestens einer Verbindung nach einem der Ansprüche 1 bis 6, eines Arzneimittels nach Anspruch 10 oder eines nach Anspruch 9 erhaltenen Arzneimittels zur Herstellung eines Arzneimittels zur Bekämpfung von bakteriellen Infektionen in Menschen und Tieren.

## Claims

1. Compound of formula 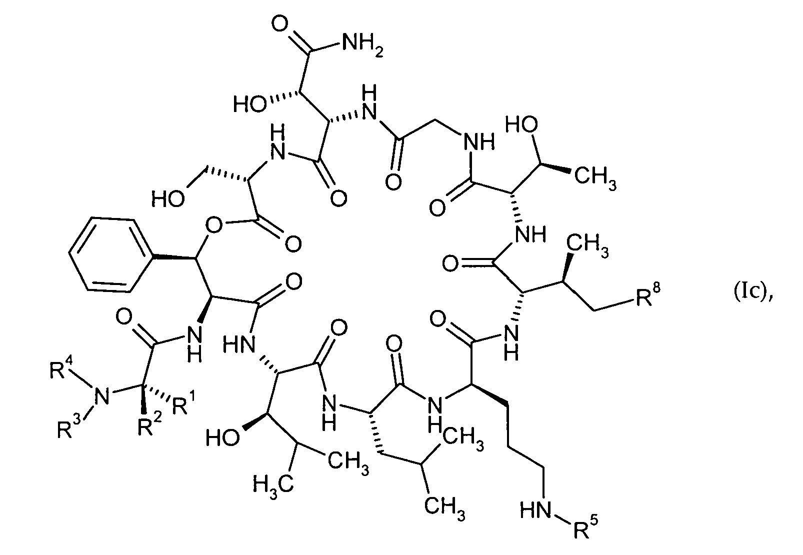 in which
R¹ represents hydrogen, C₁-C₆-alkyl, C₂-C₆-alkenyl, C₃-C₆-cycloalkyl or C₆-C₁₀-aryl,
whereby alkyl, alkenyl, cycloalkyl and aryl can be substituted with 0, 1, 2 or 3 substituents selected independently of one another from the group consisting of halogen, hydroxy, amino, cyano, trimethylsilyl, C₁-C₆-alkyl, C₁-C₆-alkoxy, benzyloxy, C₃-C₆-cycloalkyl, C₆-C₁₀-aryl, 5- to 7-membered heterocyclyl, 5- to 10-membered heteroaryl, C₁-C₆-alkylamino, C₆-C₁₀-arylamino, C₁-C₆-alkylcarbonylamino, C₆-C₁₀-arylcarbonylamino, C₁-C₆-alkylcarbonyl, C₁-C₆-alkoxycarbonyl, C₆-C₁₀-arylcarbonyl and benzyloxycarbonylamino,
wherein cycloalkyl, aryl, heterocyclyl and heteroaryl for their part can be substituted with 0, 1, 2 or 3 substituents selected independently of one another from the group consisting of halogen, hydroxy, amino, cyano, nitro, trifluoromethyl, C₁-C₆-alkyl, C₁-C₆-alkoxy, phenyl and 5- to 7-membered heterocyclyl,
R² represents hydrogen or C₁-C₄-alkyl,
R³ represents C₁-C₆-alkyl, C₃-C₆-cycloalkyl, 5- to 7-membered heterocyclyl, C₆-C₁₀-aryl, 5- or 6-membered heteroaryl, C₁-C₆-alkylcarbonyl, C₁-C₆-alkoxycarbonyl, C₃-C₆-cycloalkylcarbonyl, 5- to 7-membered heterocyclylcarbonyl, C₆-C₁₀-arylcarbonyl, 5- or 6-membered heteroarylcarbonyl or C₁-C₆-alkylaminocarbonyl,
whereby alkyl, cycloalkyl, heterocyclyl, aryl, heteroaryl, alkoxycarbonyl, cycloalkylcarbonyl, heterocyclylcarbonyl, arylcarbonyl, heteroarylcarbonyl and alkylaminocarbonyl can be substituted with 0, 1, 2 or 3 substituents selected independently of one another from the group consisting of halogen, hydroxy, amino, C₁-C₆-alkylamino and phenyl,
and
whereby alkylcarbonyl is substituted with a substituent amino or C₁-C₆-alkylamino,
and
whereby alkylcarbonyl can be substituted with a further 0, 1 or 2 substituents selected independently of one another from the group consisting of halogen, hydroxy, trimethylsilyl, C₁-C₆-alkoxy, C₁-C₆-alkylthio, benzyloxy, C₃-C₆-cycloalkyl, phenyl, naphthyl, 5- to 10-membered heteroaryl, C₁-C₆-alkylcarbonylamino, C₁-C₆-alkoxycarbonylamino, C₆-C₁₀-arylcarbonylamino, C₆-C₁₀-arylcarbonyloxy, benzyloxycarbonyl and benzyloxycarbonylamino,
wherein phenyl and heteroaryl for their part can be substituted with 0, 1, 2 or 3 substituents selected independently of one another from the group consisting of halogen, hydroxy, nitro, C₁-C₆-alkyl, C₁-C₆-alkoxy and phenyl,
R⁴ represents hydrogen, C₁-C₄-alkyl, cyclopropyl or cyclopropylmethyl,
R⁵ represents a group of formula 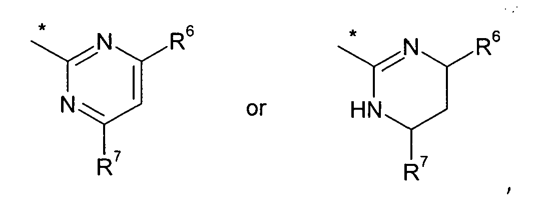 whereby
* is the linkage site to the nitrogen atom,
R⁶ and R⁷ independently of one another represent C₁-C₆-alkyl or trifluoromethyl,
R⁸ represents hydrogen or methyl,
or one of its salts, its solvates or the solvates of its salts.

2. Compound according to claim 1, **characterized in that** it corresponds to formula 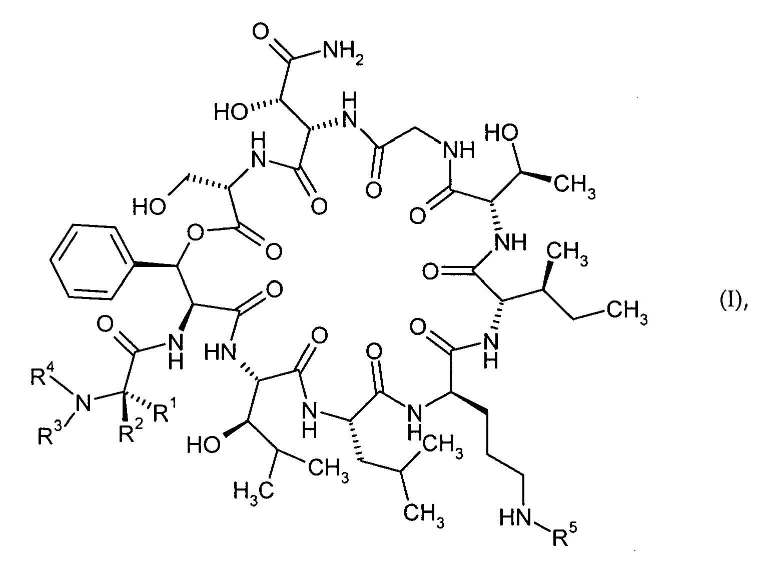 in which
R¹ represents hydrogen, C₁-C₆-alkyl, C₂-C₆-alkenyl, C₃-C₆-cycloalkyl or C₆-C₁₀-aryl,
whereby alkyl, alkenyl, cycloalkyl and aryl can be substituted with 0, 1, 2 or 3 substituents selected independently of one another from the group consisting of halogen, hydroxy, amino, cyano, trimethylsilyl, C₁-C₆-alkyl, C₁-C₆-alkoxy, benzyloxy, C₃-C₆-cycloalkyl, C₆-C₁₀-aryl, 5- to 7-membered heterocyclyl, 5- to 10-membered heteroaryl, C₁-C₆-alkylamino, C₆-C₁₀-arylamino, C₁-C₆-alkylcarbonylamino, C₆-C₁₀-arylcarbonylamino, C₁-C₆-alkylcarbonyl, C₁-C₆-alkoxycarbonyl, C₆-C₁₀-arylcarbonyl and benzyloxycarbonylamino,
wherein cycloalkyl, aryl, heterocyclyl and heteroaryl for their part can be substituted with 0, 1, 2 or 3 substituents selected independently of one another from the group consisting of halogen, hydroxy, amino, cyano, nitro, trifluoromethyl, C₁-C₆-alkyl, C₁-C₆-alkoxy, phenyl and 5- to 7-membered heterocyclyl,
R² represents hydrogen or C₁-C₄-alkyl,
R³ represents C₁-C₆-alkyl, C₃-C₆-cycloalkyl, 5- to 7-membered heterocyclyl, C₆-C₁₀-aryl, 5- or 6-membered heteroaryl, C₁-C₆-alkylcarbonyl, C₁-C₆-alkoxycarbonyl, C₃-C₆-cycloalkylcarbonyl, 5- to 7-membered heterocyclylcarbonyl, C₆-C₁₀-arylcarbonyl, 5- or 6-membered heteroarylcarbonyl or C₁-C₆-alkylaminocarbonyl,
whereby alkyl, cycloalkyl, heterocyclyl, aryl, heteroaryl, alkoxycarbonyl, cycloalkylcarbonyl, heterocyclylcarbonyl, arylcarbonyl, heteroarylcarbonyl and alkylaminocarbonyl can be substituted with 0, 1, 2 or 3 substituents selected independently of one another from the group consisting of halogen, hydroxy, amino, C₁-C₆-alkylamino and phenyl,
and
whereby alkylcarbonyl is substituted with a substituent amino or C₁-C₆-alkylamino,
and
whereby alkylcarbonyl can be substituted with a further 0, 1 or 2 substituents selected independently of one another from the group consisting of halogen, hydroxy, trimethylsilyl, C₁-C₆-alkoxy, C₁-C₆-alkylthio, benzyloxy, C₃-C₆-cycloalkyl, phenyl, naphthyl, 5- to 10-membered heteroaryl, C₁-C₆-alkylcarbonylamino, C₁-C₆-alkoxycarbonylamino, C₆-C₁₀-arylcarbonylamino, C₆-C₁₀-arylcarbonyloxy, benzyloxycarbonyl and benzyloxycarbonylamino,
wherein phenyl and heteroaryl for their part can be substituted with 0, 1, 2 or 3 substituents selected independently of one another from the group consisting of halogen, hydroxy, nitro, C₁-C₆-alkyl, C₁-C₆-alkoxy and phenyl,
R⁴ represents hydrogen, C₁-C₄-alkyl, cyclopropyl or cyclopropylmethyl,
R⁵ represents a group of formula 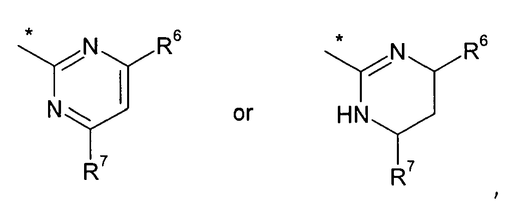 whereby
* is the linkage site to the nitrogen atom,
R⁶ and R⁷ independently of one another represent C₁-C₆-alkyl or trifluoromethyl,
or one of its salts, its solvates or of the solvates of its salts.

3. Compound according to claim 2, **characterized in that**
R¹ represents 2-methylprop-1-yl, 2,2-dimethylprop-1-yl, 2,2-dimethylbut-1-yl, 1-trimethylsilylmethyl, 2-trimethylsilyleth-1-yl, 1-hydroxy-2-methylprop-1-yl, 1-hydroxy-2,2-dimethylprop-1-yl, 1-hydroxy-2,2-dimethylbut-1-yl, 1-hydroxy-2-ethyl-2-methylbut-1-yl, 1-hydroxy-2,2-diethylbut-1-yl, phenylmethyl, 1-hydroxy-1-phenylmethyl, 2-pyridylmethyl or 3-pyridylmethyl,
whereby 2-pyridylmethyl or 3-pyridylmethyl can be substituted with 0, 1, 2 or 3 substituents selected independently of one another from the group consisting of hydroxy, amino, trifluoromethyl, methyl, methoxy and morpholinyl,
R² represents hydrogen,
R³ represents 1-amino-3-methylbut-1-ylcarbonyl, 1-amino-3,3-dimethylbut-1-ylcarbonyl or 1-amino-2-trimethylsilyleth-1-ylcarbonyl,
R⁴ represents hydrogen,
R⁵ represents a group of formula 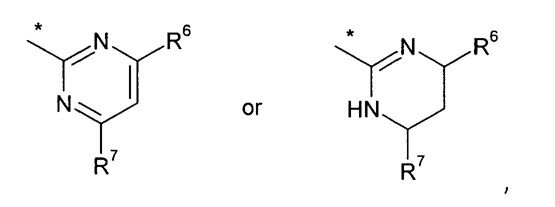 whereby
* is the linkage site to the nitrogen atom,
R⁶ and R⁷ independently of one another represent C₁-C₆-alkyl or trifluoromethyl,
or one of its salts, its solvates or the solvates of its salts.

4. Compound according to one of claims 2 or 3, **characterized in that**
R¹ represents 2-methylprop-l-yl,
R² represents hydrogen,
R³ represents 1-amino-3-methylbut-1-ylcarbonyl,
R⁴ represents hydrogen,
R⁵ represents a group of formula 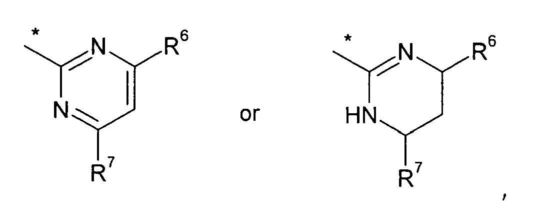 whereby
* is the linkage site to the nitrogen atom,
R⁶ and R⁷ independently of one another represent C₁-C₆-alkyl,
or one of its salts, its solvates or the solvates of its salts.

5. Compound according to one of claims 2 or 3, **characterized in that**
R¹ represents 2,2-dimethylprop-1-yl,
R² represents hydrogen,
R³ represents 1-amino-3,3-dimethylbut-1-ylcarbonyl,
R⁴ represents hydrogen,
R⁵ represents a group of formula 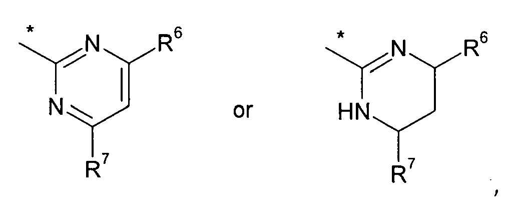 whereby
* is the linkage site to the nitrogen atom,
R⁶ and R⁷ independently of one another represent C₁-C₆-alkyl,
or one of its salts, its solvates or the solvates of its salts.

6. Compound according to one of claims 2 or 3, **characterized in that**
R¹ represents 2,2-dimethylprop-1-yl, 1-trimethylsilylmethyl or 3-pyridylmethyl,
whereby 3-pyridylmethyl can be substituted with a substituent trifluoromethyl,
R² represents hydrogen,
R³ represents 1-amino-3,3-dimethylbut-1-ylcarbonyl or 1-amino-2-trimethylsilyleth-1-ylcarbonyl,
R⁴ represents hydrogen,
R⁵ represents a group of formula 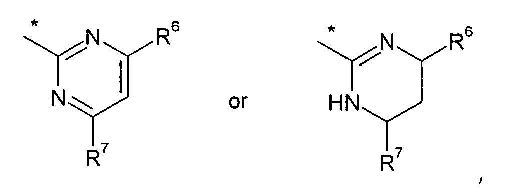 whereby
* is the linkage site to the nitrogen atom,
R⁶ and R⁷ independently of one another represent C₁-C₆-alkyl or trifluoromethyl,
or one of its salts, its solvates or the solvates of its salts.

7. Method for preparing a compound of formula (Ic) according to claim 1, **characterized in that**
according to method [A], a compound of formula 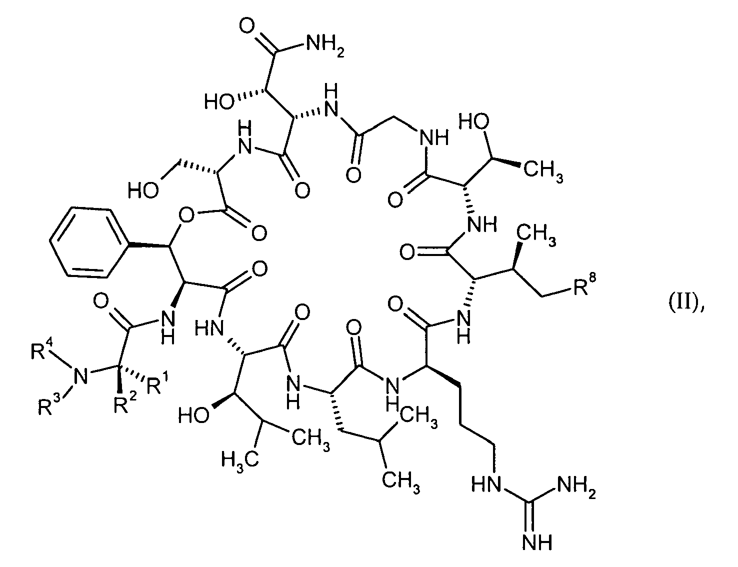 in which R¹, R², R³, R⁴ and R⁸ have the meaning indicated in claim 1,
is reacted with a compound of formula 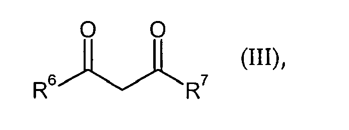 in which R⁶ and R⁷ have the meaning indicated in claim 1,
to give a compound of formula 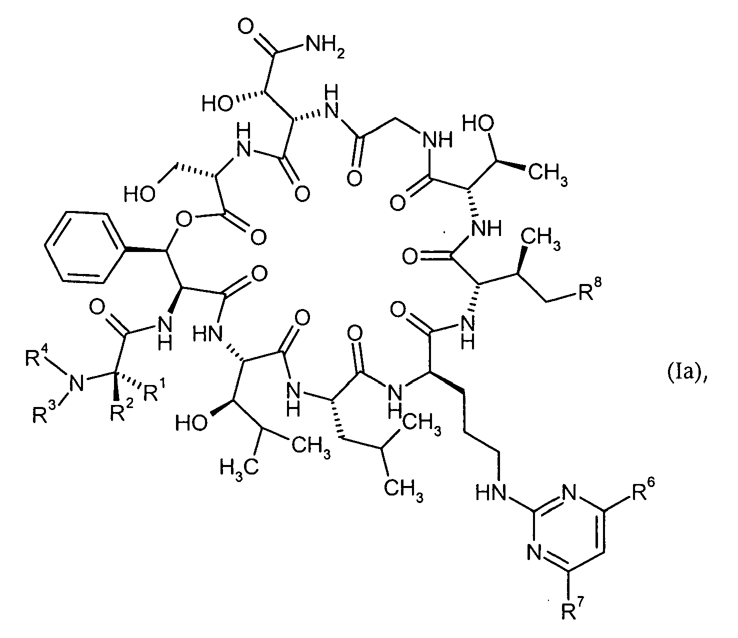 in which R¹, R², R³, R⁴, R⁶, R⁷ and R⁸ have the meaning indicated in claim 1,
or
according to method [B], a compound of formula (Ia) is reacted with a reducing agent to give a compound of formula 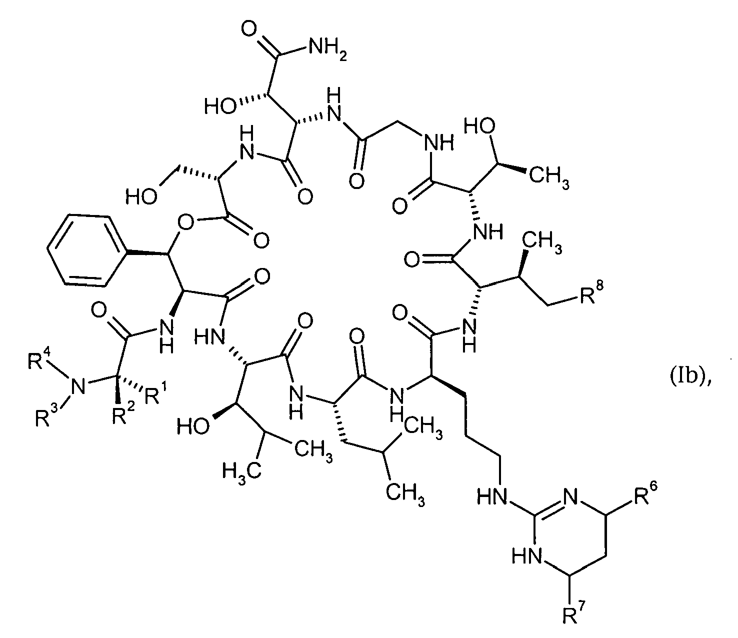 in which R¹, R², R³, R⁴, R⁶, R⁷ and R⁸ have the meaning indicated in claim 1.

8. Compound according to one of claims 1 to 6 for the treatment and/or prophylaxis of diseases.

9. Use of a compound according to one of claims 1 to 6 for the production of a medicament for the treatment and/or prophylaxis of bacterial infections.

10. Medicament comprising a compound according to one of claims 1 to 6 in combination with an inert, non-toxic, pharmaceutically acceptable excipient.

11. Medicament according to claim 10 for the treatment and/or prophylaxis of bacterial infections.

12. Use of an antibacterially effective amount of at least one compound according to one of claims 1 to 6, of a medicament according to claim 10 or of a medicament obtained according to claim 9 for the production of a medicament for controlling bacterial infections in humans and animals.

## Revendications

1. Composé de formule 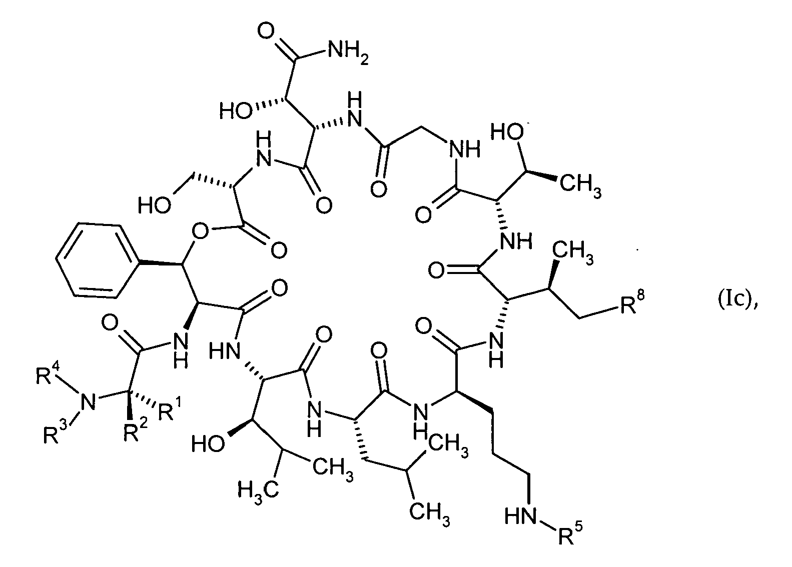 dans laquelle
R¹ représente l'hydrogène, un reste alkyle en C₁ à C₆, alcényle en C₂ à C₆, cycloalkyle en C₃ à C₆ ou aryle en C₆ à C10,
les restes alkyle, alcényle, cycloalkyle et aryle pouvant être substitués avec 0, 1, 2 ou 3 substituants choisis, indépendemment les uns des autres, dans le groupe constitué d'halogène, hydroxy, amino, cyano, triméthylsilyle, alkyle en C₁ à C₆, alkoxy en C₁ à C₆, benzyloxy, cycloalkyle en C₃ à C₆, aryle en C₆ à C₁₀, hétérocyclyle pentagonal à heptagonal, hétéroaryle pentagonal à décagonal, alkylamino en C₁ à C₆, arylamino en C₆ à C₁₀, (alkyle en C₁ à C₆)carbonylamino, (aryle en C₆ à C₁₀)carbonylamino, (alkyle en C₁ à C₆)carbonyle, (alkoxy en C₁ à C₆)carbonyle, (aryle en C₆ à C₁₀)carbonyle et benzyloxycarbonylamino,
où les restes cycloalkyle, aryle, hétérocyclyle et hétéroaryle peuvent eux-mêmes être substitués avec 0, 1, 2 ou 3 substituants choisis, indépendamment les uns des autres, dans le groupe constitué d'halogène, hydroxy, amino, cyano, nitro, trifluorométhyle, alkyle en C₁ à C₆, alkoxy en C₁ à C₆, phényle et hétérocyclyle pentagonal à heptagonal,
R² représente l'hydrogène ou un reste alkyle en C₁ à C₄,
R³ représente un reste alkyle en C₁ à C₆, cycloalkyle en C₃ à C₆, hétérocyclyle pentagonal à heptagonal, aryle en C₆ à C₁₀, hétéroaryle pentagonal ou hexagonal, (alkyle en C₁ à C₆)carbonyle, (alkoxy en C₁ à C₆)carbonyle, (cycloalkyle en C₃ à C₆)carbonyle, (hétérocyclyle pentagonal à heptagonal)carbonyle, (aryle en C₆ à C₁₀)carbonyle, (hétéroaryle pentagonal ou hexagonal)carbonyle ou (alkylamino en C₁ à C₆)carbonyle,
les restes alkyle, cycloalkyle, hétérocyclyle, aryle, hétéroaryle, alkoxycarbonyle, cycloalkylcarbonyle, hétérocyclylcarbonyle, arylcarbonyle, hétéroarylcarbonyle et alkylaminocarbonyle pouvant être substitués avec 0, 1, 2 ou 3 substituants choisis, indépendamment les uns des autres, dans le groupe constitué d'halogène, hydroxy, amino, alkylamino en C₁ à C₆ et phényle,
et
le reste alkylcarbonyle étant substitué avec un substituant amino ou alkylamino en C₁ à C₆,
et
le reste alkylcarbonyle pouvant être substitué avec 0, 1 ou 2 autres substituants choisis, indépendamment l'un de l'autre, dans le groupe constitué d'halogène, hydroxy, triméthylsilyle, alkoxy en C₁ à C₆, alkylthio en C₁ à C₆, benzyloxy, cycloalkyle en C₃ à C₆, phényle, naphtyle, hétéroaryle pentagonal à décagonal, (alkyle en C₁ à C₆)carbonylamino, (alkoxy en C₁ à C₆)carbonylamino, (aryle en C₆ à C₁₀)carbonylamino, (aryle en C₆ à C₁₀)carbonyloxy, benzyloxycarbonyle et benzyloxycarbonylamino,
où les restes phényle et hétéroaryle peuvent eux-mêmes être substitués avec 0, 1, 2 ou 3 substituants choisis, indépendamment les uns des autres, dans le groupe constitué d'halogène, hydroxy, nitro, alkyle en C₁ à C₆, alkoxy en C₁ à C₆ et phényle,
R⁴ représente l'hydrogène, un reste alkyle en C₁ à C₄, cyclopropyle ou cyclopropylméthyle,
R⁵ est un groupe de formule 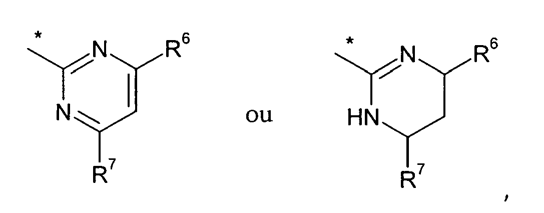
* indiquant la position de rattachement à l'atome d'azote,
R⁶ et R⁷ représentant, indépendamment l'un de l'autre, un reste alkyle en C₁ à C₆ ou trifluorométhyle,
R⁸ représente l'hydrogène ou un reste méthyle,
ou l'un de ses sels, de ses produits de solvatation ou des produits de solvatation de ses sels.

2. Composé suivant la revendication 1, **caractérisé en ce qu'**il répond à la formule 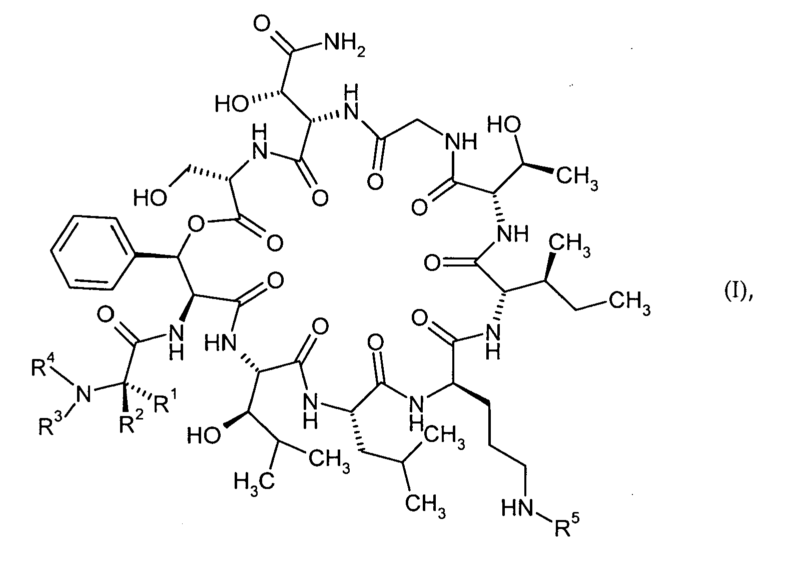 dans laquelle
R¹ représente l'hydrogène, un reste alkyle en C₁ à C₆, alcényle en C₂ à C₆, cycloalkyle en C₃ à C₆ ou aryle en C₆ à C₁₀,
les restes alkyle, alcényle, cycloalkyle et aryle pouvant être substitués avec 0, 1, 2 ou 3 substituants choisis, indépendamment les uns des autres, dans le groupe constitué d'halogène, hydroxy, amino, cyano, triméthylsilyle, alkyle en C₁ à C₆, alkoxy en C₁ à C₆, benzyloxy, cycloalkyle en C₃ à C₆, aryle en C₆ à C₁₀, hétérocyclyle pentagonal à heptagonal, hétéroaryle pentagonal à décagonal, alkylamino en C₁ à C₆, arylamino en C₆ à C₁₀, (alkyle en C₁ à C₆)carbonylamino, (aryle en C₆ à C₁₀)carbonylamino, (alkyle en C₁ à C₆)carbonyle, (alkoxy en C₁ à C₆)carbonyle, (aryle en C₆ à C₁₀)carbonyle et benzyloxycarbonylamino,
où les restes cycloalkyle, aryle, hétérocyclyle et hétéroaryle peuvent eux-mêmes être substitués avec 0, 1, 2 ou 3 substituants choisis, indépendamment les uns des autres, dans le groupe constitué d'halogène, hydroxy, amino, cyano, nitro, trifluorométhyle, alkyle en C₁ à C₆, alkoxy en C₁ à C₆, phényle et hétérocyclyle pentagonal à heptagonal,
R² représente l'hydrogène ou un reste alkyle en C₁ à C₄,
R³ représente un reste alkyle en C₁ à C₆, cycloalkyle en C₃ à C₆, hétérocyclyle pentagonal à heptagonal, aryle en C₆ à C₁₀, hétéroaryle pentagonal ou hexagonal, (alkyle en C₁ à C₆)carbonyle, (alkoxy en C₁ à C₆)carbonyle, (cycloalkyle en C₃ à C₆)carbonyle, (hétérocyclyle pentagonal à heptagonal)carbonyle, (aryle en C₆ à C₁₀)carbonyle, (hétéroaryle pentagonal ou hexagonal)carbonyle ou (alkyle en C₁ à C₆)aminocarbonyle,
les restes alkyle, cycloalkyle, hétérocyclyle, aryle, hétéroaryle, alkoxycarbonyle, cycloalkylcarbonyle, hétérocyclylcarbonyle, arylcarbonyle, hétéroarylcarbonyle et alkylaminocarbonyle pouvant être substitués avec 0, 1, 2 ou 3 substituants choisis, indépendamment les uns des autres, dans le groupe constitué d'halogène, hydroxy, amino, alkylamino en C₁ à C₆ et phényle,
et
le reste alkylcarbonyle étant substitué avec un substituant amino ou alkylamino en C₁ à C₆,
et
le reste alkylcarbonyle pouvant être substitué avec 0, 1 ou 2 autres substituants choisis, indépendamment l'un de l'autre, dans le groupe constitué d'halogène, hydroxy, triméthylsilyle, alkoxy en C₁ à C₆, alkylthio en C₁ à C₆, benzyloxy, cycloalkyle en C₃ à C₆, phényle, naphtyle, hétéroaryle pentagonal à décagonal, (alkyle en C₁ à C₆)carbonylamino, (alkoxy en C₁ à C₆)carbonylamino, (aryle en C₆ à C₁₀)carbonylamino
(aryle en C₆ à C₁₀)carbonyloxy, benzyloxycarbonyle et benzyloxycarbonylamino,
où les restes phényle et hétéroaryle peuvent eux-mêmes être substitués avec 0, 1, 2 ou 3 substituants choisis, indépendamment les uns des autres, dans le groupe constitué d'halogène, hydroxy, nitro, alkyle en C₁ à C₆, alkoxy en C₁ à C₆ et phényle,
R⁴ représente l'hydrogène, un reste alkyle en C₁ à C₄, cyclopropyle ou cyclopropylméthyle,
R⁵ est un groupe de formule 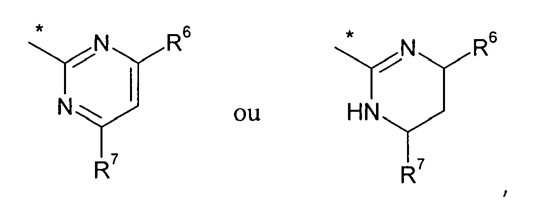
* indiquant la position de rattachement à l'atome d'azote,
R⁶ et R⁷ représentant, indépendamment l'un de l'autre, un reste alkyle en C₁ à C₆ ou trifluorométhyle,
ou l'un de ses sels, de ses produits de solvatation ou des produits de solvatation de ses sels.

3. Composé suivant la revendication 2, **caractérisé en ce que**
R¹ est un reste 2-méthylprop-1-yle, 2,2-diméthylprop-1-yle, 2,2-diméthylbut-1-yle, 1-triméthylsilylméthyle, 2-triméthylsilyléth-1-yle, 1-hydroxy-2-méthylprop-1-yle, 1-hydroxy-2,2-diméthylprop-1-yle, 1-hydroxy-2,2-diméthylbut-1-yle, 1-hydroxy-2-éthyl-2-méthylbut-1-yle, 1-hydroxy-2,2-diéthylbut-1-yle, phénylméthyle, 1-hydroxy-1-phénylméthyle, 2-pyridylméthyle ou 3-pyridylméthyle,
les restes 2-pyridylméthyle ou 3-pyridylméthyle pouvant être substitués avec 0, 1, 2 ou 3 substituants choisis, indépendamment les uns des autres, dans le groupe constitué d'hydroxy, amino, trifluorométhyle, méthyle, méthoxy et morpholinyle,
R² représente l'hydrogène,
R³ est un reste 1-amino-3-méthylbut-1-ylcarbonyle, 1-amino-3,3-diméthylbut-1-ylcarbonyle ou 1-amino-2-triméthylsilyléth-1-ylcarbonyle,
_{R}⁴ représente l'hydrogène,
R⁵ est un groupe de formule 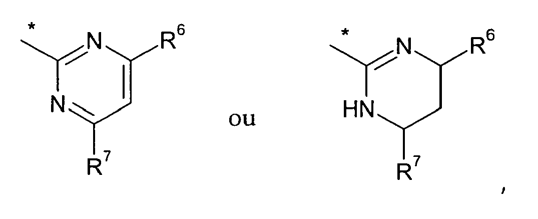
* indiquant la position de rattachement à l'atome d'azote,
R⁶ et R⁷ représentant, indépendamment l'un de l'autre, un reste alkyle en C₁ à C₆ ou trifluorométhyle,
ou l'un de ses sels, de ses produits de solvatation ou des produits de solvatation de ses sels.

4. Composé suivant l'une des revendications 2 ou 3, **caractérisé en ce que**
R¹ est un reste 2-méthylprop-1-yle,
R² représente l'hydrogène,
R³ est un reste 1-amino-3-méthylbut-1-ylcarbonyle,
R⁴ représente l'hydrogène,
R⁵ est un groupe de formule 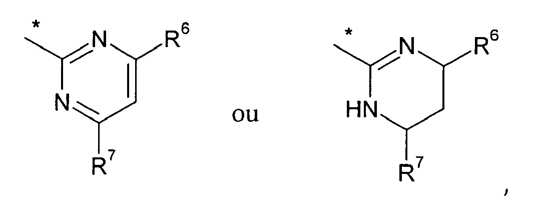
* indiquant la position de rattachement à l'atome d'azote,
R⁶ et R⁷ représentant, indépendamment l'un de l'autre, un reste alkyle en C₁ à C₆,
ou l'un de ses sels, de ses produits de solvatation ou des produits de solvatation de ses sels.

5. Composé suivant l'une des revendications 2 ou 3, **caractérisé en ce que**
R¹ est un reste 2,2-diméthylprop-1-yle,
R² représente l'hydrogène,
R³ est un reste 1-amino-3,3-diméthylbut-1-ylcarbonyle,
R⁴ représente l'hydrogène,
R⁵ est un groupe de formule 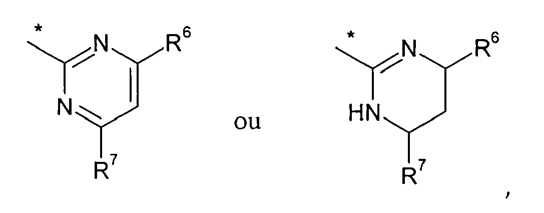
* indiquant la position de rattachement à l'atome d'azote,
R⁶ et R⁷ représentant, indépendamment l'un de l'autre, un reste alkyle en C₁ à C₆,
ou l'un de ses sels, de ses produits de solvatation ou des produits de solvatation de ses sels.

6. Composé suivant l'une des revendications 2 ou 3, **caractérisé en ce que**
R¹ est un reste 2,2-diméthylprop-1-yle, 1-triméthylsilylméthyle ou 3-pyridylméthyle,
le reste 3-pyridylméthyle pouvant être substitué avec un substituant trifluorométhyle,
R² représente l'hydrogène,
R³ est un reste 1-amino-3,3-diméthylbut-1-ylcarbonyle ou 1-amino-2-triméthylsilyléth-1-ylcarbonyle,
R⁴ représente l'hydrogène,
R⁵ est un groupe de formule 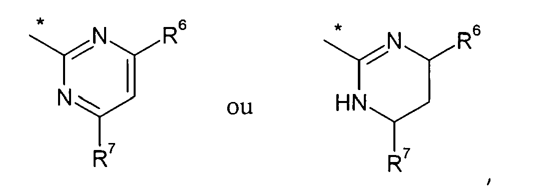
* indiquant la position de rattachement à l'atome d'azote,
R⁶ et R⁷ représentant indépendamment l'un de l'autre un reste alkyle en C₁ à C₆ ou trifluorométhyle,
ou l'un de ses sels, de ses produits de solvatation ou des produits de solvatation de ses sels.

7. Procédé de production d'un composé de formule (Ic) suivant la revendication 1, **caractérisé en ce que**
selon le procédé [A], un composé de formule 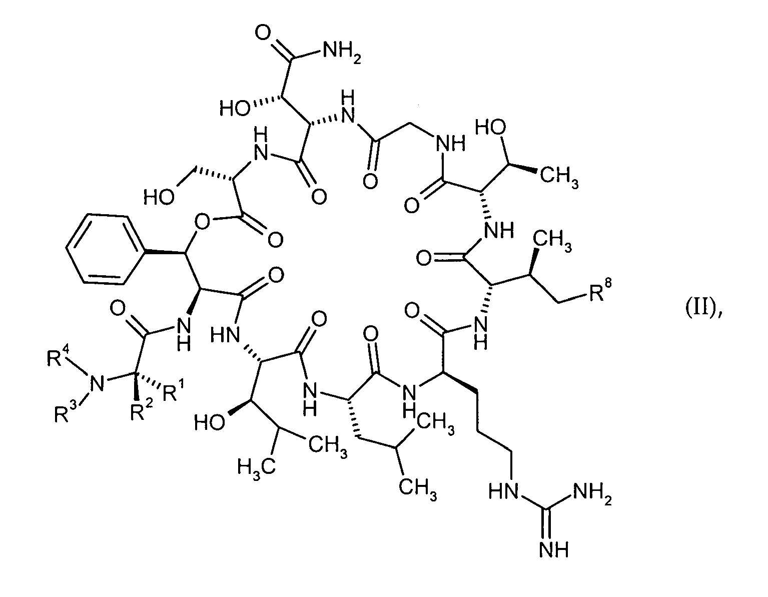 dans laquelle R¹, R², R³, R⁴, et R⁸ ont la définition indiquée dans la revendication 1,
est amené à réagir avec un composé de formule 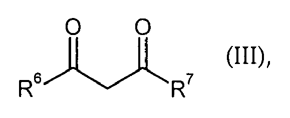 dans laquelle R⁶ et R⁷ ont la définition indiquée dans la revendication 1,
pour produire un composé de formule 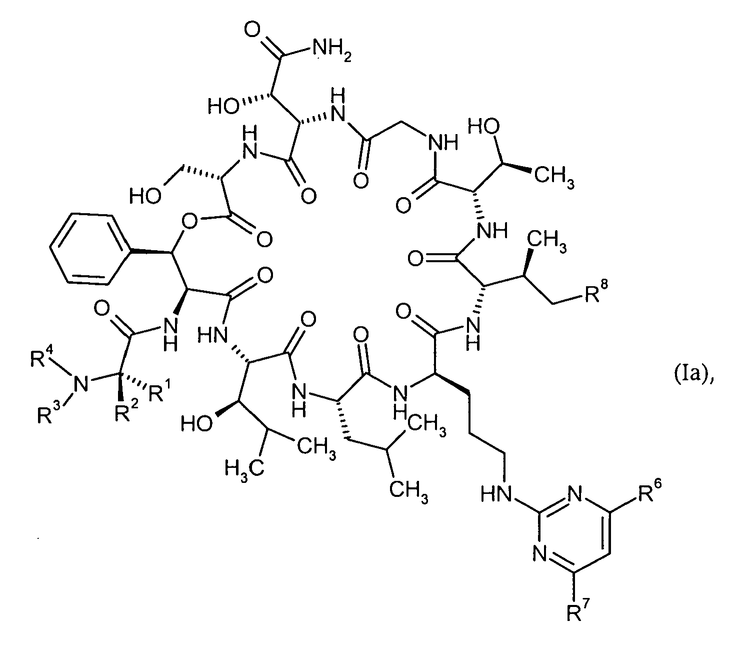 dans laquelle R¹, R², R³, R⁴, R⁶, R⁷ et R⁸ ont la définition indiquée dans la revendication 1,
ou bien
selon le procédé [B], un composé de formule (Ia) est amené à réagir avec un agent réducteur pour produire un composé de formule 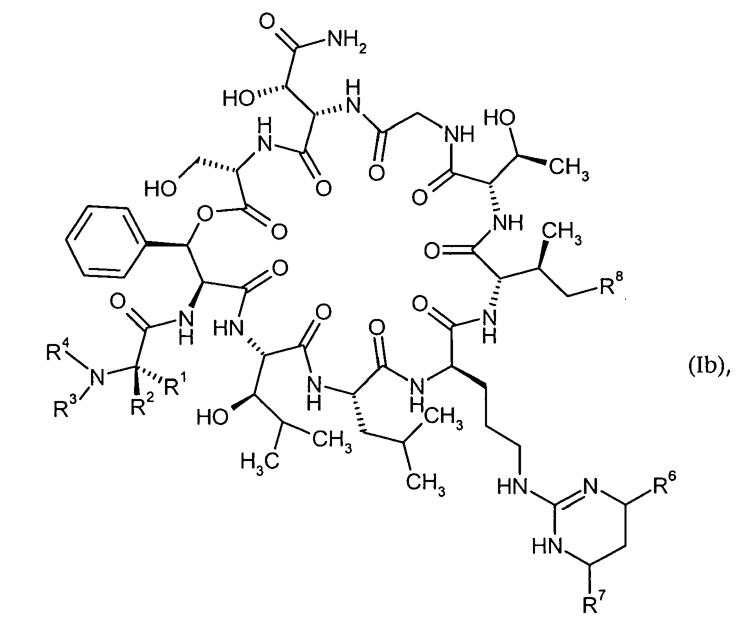 dans laquelle R¹, R², R³, R⁴, R⁶, R⁷ et R⁸ ont la définition indiquée dans la revendication 1.

8. Composé suivant l'une des revendications 1 à 6, destiné au traitement et/ou à la prophylaxie de maladies.

9. Utilisation d'un composé suivant l'une des revendications 1 à 6 pour la préparation d'un médicament destiné au traitement et/ou à la prophylaxie d'infections bactériennes.

10. Médicament contenant un composé suivant l'une des revendications 1 à 6 en combinaison avec une substance auxiliaire inerte non toxique pharmaceutiquement acceptable.

11. Médicament suivant la revendication 10, destiné au traitement et/ou à la prophylaxie d'infections bactériennes.

12. Utilisation d'une quantité à effet antibactérien d'au moins un composé suivant l'une des revendications 1 à 6, d'un médicament suivant la revendication 10 ou d'un médicament obtenu suivant la revendication 9 pour la préparation d'un médicament destiné à combattre des infections bactériennes chez l'homme et chez des animaux.
